Europäisches Patentamt
European Patent Office
Office européen des brevets

(19)

(11) Publication number: **0 381 374 A1**

(12) **EUROPEAN PATENT APPLICATION**

(21) Application number: **90300778.9**

(22) Date of filing: **25.01.90**

(51) Int. Cl.[5]: **C07D 417/04, C07D 413/04, C07D 403/04, A61K 31/535, A61K 31/53, A61K 31/54**

(30) Priority: **27.01.89 GB 8901836**

(43) Date of publication of application: **08.08.90 Bulletin 90/32**

(84) Designated Contracting States: **CH DE FR GB IT LI NL**


(71) Applicant: **LES LABORATOIRES BEECHAM S.A.**
**13-15-25 Boulevard de l'Amiral Bruix**
**F-75782 Paris Cédex 16(FR)**

(72) Inventor: **Nadler, Guy Les Laboratoires Beecham S.A.**
**4 Rue Du Chesnay Beauregard**
**F-35760 Saint-Gregoire, Rennes(FR)**
Inventor: **Martin, Michel Les Laboratoires Beecham S.A.**
**4 Rue Du Chesnay Beauregard**
**F-35760 Saint-Gregoire, Rennes(FR)**
Inventor: **Zimmermann, Richard Les Laboratoires Beecham S.A**
**4 Rue Du Chesnay Beauregard**
**F-35760 Saint-Gregoire, Rennes(FR)**

(74) Representative: **Russell, Brian John et al**
**Beecham Pharmaceuticals Great Burgh Yew Tree Bottom Road**
**Epsom Surrey, KT18 5XQ(GB)**


(54) **Phosphodiesterase inhibitors.**


(57) A compound of formula (I), or a pharmaceutically acceptable salt thereof:

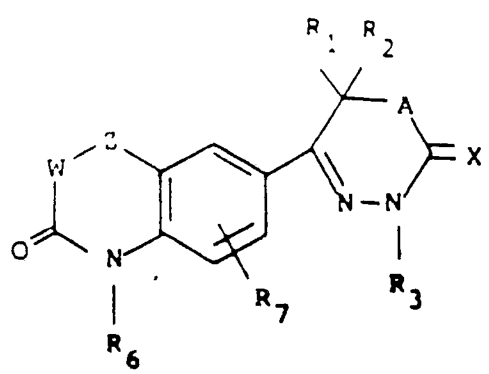

(I)

in which,
$R_1$ is hydrogen, $C_{1-6}$ alkyl or $CH_2OR_6$;
$R_2$ is hydrogen or $C_{1-6}$ alkyl;
$R_3$ is hydrogen or $C_{1-6}$ alkyl;
each of W and Z, which are different, represents $-CR_4R_5-$ or $-(CR_xR_y)_n-$, in which,
$R_4$ is hydrogen, $C_{1-3}$ alkyl, $C_{1-3}$ alkylthio, $C_{1-3}$ alkoxy or $C_{1-6}$ alkyl phenyl;
$R_5$ is $C_{1-3}$ alkyl, $C_{1-3}$ alkylthio, $C_{1-3}$ alkoxy, phenyl, substituted phenyl, $C_{3-6}$ cycloalkyl, phenylthio, $C_{1-6}$


EP 0 381 374 A1

alkyl phenyl, halo-substituted benzyl, or heteroaryl; or together $R_4$ and $R_5$ form a 3 to 6 membered carbocyclic ring, or a heterocyclic ring containing one or two ring oxygen, nitrogen or sulphur atoms,
or $R_4$ and $R_5$ together form an oxo or methylene group;
each of $R_x$ and $R_y$ is hydrogen or $C_{1-3}$ alkyl; n is zero or 1;
$R_6$ is phenyl substituted aminocarbonyl, $C_{1-6}$ alkoxy carbonyl-$C_{1-6}$ alkyl, phenyl-$C_{1-6}$ alkyl, phenyl, $C_{3-6}$ cycloalkylcarbonyl, $C_{3-6}$ cycloalkylcarbonyl-$C_{1-6}$ alkyl, $C_{3-6}$ cycloalkyl $C_{1-6}$ alkyl;
$C_{1-6}$ alkylthiocarbonyl; halo-substituted $C_{1-6}$ alkoxycarbonyl; $C_{1-6}$ alkoxy $C_{1-6}$ alkyleneoxycarbonyl; $C_{1-6}$ alkylthio $C_{1-6}$ alkyleneoxycarbonyl; $C_{1-6}$ alkoxythiocarbonyl; $C_{3-6}$ cycloalkyloxycarbonyl; cyano substituted $C_{1-6}$ alkoxycarbonyl; di-$C_{1-6}$ alkylphosphonate; $C_{1-6}$ alkenyloxycarbonyl; or
$R_6$ is hydrogen when $R_5$ is phenyl, $C_{3-6}$ cycloalkyl, phenylthio, $C_{1-6}$ alkylphenyl or halo-substituted benzyl;
$R_6$ is benzoyl or aminobenzoyl when $R_4$ and $R_5$ form a $C_{3-6}$ cycloalkyl ring;
$R_7$ is hydrogen, $C_{1-6}$ alkyl or halogen;
X is oxygen or sulphur;
and A is sulphur, oxygen or -NH-, is useful for the treatment of heart disease.

## Phosphodiesterase Inhibitors

This invention relates to compounds having pharmacological activity, pharmaceutical compositions containing them, processes for their preparation, and their use as active therapeutic agents, particularly in the treatment of acute or chronic heart disease.

EP-A-0052442 discloses phenyl-thiadiazinone, oxadiazinone or triazinone derivatives which are phosphodiesterase inhibitors and are said to possess cardiotonic properties. There is no disclosure in this document of the derivatives having the property of increasing the sensitivity of myocardial contractile proteins to calcium, which is believed to be an additional, useful mechanism of action for cardiotonic agents.

It has now been discovered that certain novel phenyl-thiadiazinone, oxadiazinone and triazinone derivatives in which the phenyl nucleus forms part of a substituted 2-oxo-2,3-dihydroindole or 2-oxo-2,3,-dihydroquinone ring are phosphodiesterase inhibitors, and some of these derivatives may also increase the sensitivity of myocardial contractile proteins to calcium. The novel derivatives are therefore potentially valuable drugs in the treatment of congestive heart failure.

It has also been discovered that some of the derivatives have bronchodilator activity and are therefore of potential utility in the treatment of asthmatic conditions.

According to the present invention there is provided a compound of formula (I), or a pharmaceutically acceptable salt thereof:

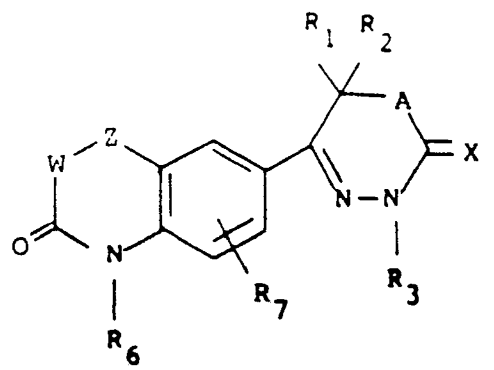

(I)

in which

$R_1$ is hydrogen, $C_{1-6}$ alkyl or $CH_2OR_6$;

$R_2$ is hydrogen or $C_{1-6}$ alkyl;

$R_3$ is hydrogen or $C_{1-6}$ alkyl;

each of W and Z, which are different, represents $-CR_4R_5-$ or $-(CR_xR_y)_n-$, in which,

$R_4$ is hydrogen, $C_{1-3}$ alkyl, $C_{1-3}$ alkylthio, $C_{1-3}$ alkoxy or $C_{1-6}$ alkyl phenyl;

$R_5$ is $C_{1-3}$ alkyl, $C_{1-3}$ alkylthio, $C_{1-3}$ alkoxy, phenyl, substituted phenyl, $C_{3-6}$ cycloalkyl, phenylthio, $C_{1-6}$ alkyl phenyl, halo-substituted benzyl, or heteroaryl; or together $R_4$ and $R_5$ form a 3 to 6 membered carbocyclic ring, or a heterocyclic ring containing one or two ring oxygen, nitrogen or sulphur atoms,

or $R_4$ and $R_5$ together form an oxo or methylene group;

each of $R_x$ and $R_y$ is hydrogen or $C_{1-3}$ alkyl; n is zero or 1;

$R_6$ is phenyl substituted aminocarbonyl, $C_{1-6}$ alkoxy carbonyl-$C_{1-6}$ alkyl, phenyl-$C_{1-6}$ alkyl, phenyl, $C_{3-6}$ cycloalkylcarbonyl, $C_{3-6}$ cycloalkylcarbonyl-$C_{1-6}$ alkyl, $C_{3-6}$ cycloalkyl $C_{1-6}$ alkyl;

$C_{1-6}$ alkylthiocarbonyl; halo-substituted $C_{1-6}$ alkoxycarbonyl; $C_{1-6}$ alkoxy $C_{1-6}$ alkyleneoxycarbonyl; $C_{1-6}$ alkylthio $C_{1-6}$ alkyleneoxycarbonyl; $C_{1-6}$ alkoxythiocarbonyl $C_{3-6}$ cycloalkyloxycarbonyl; cyano substituted $C_{1-6}$ alkoxycarbonyl; di-$C_{1-6}$ alkylphosphonate; $C_{1-6}$ alkenyloxycarbonyl; or

$R_6$ is hydrogen when $R_5$ is phenyl; $C_{3-6}$ cycloalkyl; phenylthio; $C_{1-6}$ alkylphenyl or halo-substituted benzyl;

$R_6$ is benzoyl or aminobenzoyl when $R_4$ and $R_5$ form a $C_{3-6}$ cycloalkyl ring;

$R_7$ is hydrogen, $C_{1-6}$ alkyl or halogen;

X is oxygen or sulphur;

and A is sulphur, oxygen or -NH-.

Preferably, n is zero and/or X is oxygen and/or A is sulphur.

When $R_4$ and $R_5$ form a carbocyclic or heterocyclic ring, the ring may be saturated or unsaturated and

substituted or unsubstituted. When the heterocyclic ring is substituted, suitable substituents include one or more oxo groups.

Preferred $C_{1-6}$ alkyl groups in $R_6$ are methyl and ethyl; preferred $C_{1-6}$ alkoxy groups in $R_6$ are methoxy and ethoxy; and the preferred halogen in $R_6$ is fluoro. When $R_6$ is halo substituted $C_{1-6}$ alkoxy carbonyl, it is preferably a trifluoro alkoxy group.

The term 'heteroaryl' is used herein to include single or fused ring systems having 5 to 12 ring atoms, and comprising up to four hetero-atoms in the or each ring selected from oxygen, nitrogen and sulphur.

Preferred $R_1$ to $R_7$ substituents may be listed as follows:

$R_1$ is hydrogen or methyl;
$R_2$ is hydrogen or methyl;
$R_3$ is hydrogen or methyl;
$R_4$ is hydrogen or methyl;
$R_5$ is methyl or phenyl; or
$R_4$ and $R_5$ together are cyclopropyl or cyclopentyl;
$R_6$ is hydrogen, p-aminobenzoyl, phenylaminocarbonyl, ethoxycarbonylmethyl, benzyl, benzoyl, phenyl, phenyl-ethyl, or cyclohexyl-methyl;
and $R_7$ is hydrogen.

Preferably, when n is 1, each of $R_x$ and $R_y$ is hydrogen; or each of $R_x$ and $R_y$ is hydrogen or methyl when $R_4$ and $R_5$ are hydrogen.

Particular compounds of the invention are:

1′-[(4-Aminophenyl)carbonyl]-5′-(3,6-dihydro-6-methyl-2-oxo-2H-1,3,4-thiadiazin-5-yl)-spiro[cyclopentane-1,3′-[3H]-indol]-2′(1′H)-one, hydrochloride

2,3-Dihydro-5-(3,6-dihydro-6-methyl-2-oxo-2H-1,3,4-thiadiazin-5-yl)-3,3-dimethyl-2-oxo-N-phenyl-1H-indole-1-carboxamide

Ethyl 2,3-dihydro-5-(3,6-dihydro-6-methyl-2-oxo-2H-1,3,4-thiadiazin-5-yl)-3,3-dimethyl-2-oxo-N-1H-indole-1-acetate

1,3-Dihydro-5-(3,6-dihydro-6-methyl-2-oxo-2H-1,3,4-thiadiazin-5-yl)-3-phenyl-2H-indol-2-one

1,3-Dihydro-5-(3,6-dihydro-6-methyl-2-oxo-2H-1,3,4-thiadiazin-5-yl)-3,3-dimethyl-1-(phenylmethyl)-2H-indol-2-one

1′-Benzoyl-5′-(3,6-dihydro-6-methyl-2-oxo-2H-1,3,4-thiadiazin-5-yl)-spiro[cyclopropane-1,3′-[3H]indol]-2′-(1H)-one

1,3-Dihydro-5-(3,6-dihydro-6-methyl-2-oxo-2H-1,3,4-thiadiazin-5-yl)-3,3-dimethyl-1-phenyl-2H-indol-2-one

1,3-Dihydro-5-(3,6-dihydro-6-methyl-2-oxo-2H-1,3,4-thiadiazin-5-yl)-3,3-dimethyl-1-(2-phenylethyl)-2H-indol-2-one

1-Cyclohexylmethyl-5-(3,6-dihydro-6-methyl-2-oxo-2H-1,3,4-thiadiazin-5-yl)-1,3-dihydro-3,3-dimethyl-2H-indol-2-one.

3-Cyclohexyl-1,3-dihydro-5-(3,6-dihydro-6-methyl-2-oxo-2H-1,3,4-thiadiazin-5-yl)-2H-indol-2-one

1,3-Dihydro-5-(3,6-dihydro-2-oxo-2H-1,3,4-thiadiazin-5-yl)-3-phenylthio-2H-indol-2-one

3-Cyclohexyl-1,3-dihydro-5-(3,6-dihydro-2-oxo-2H-1,3,4-thiadiazin-5-yl)-2H-indol-2-one

1,3-Dihydro-5-(3,6-dihydro-6-methyl-2-oxo-2H-1,3,4-thiadiazin-5-yl)-3-methyl-3-phenylthio-2H-indol-2-one

1,3-Dihydro-5-(3,6-dihydro-6-methyl-2-oxo-2H-1,3,4-thiadiazin-5-yl)-3,3-di-(4-methylphenyl)-2H-indol-2-one

S-Ethyl 2,3-dihydro-5-(3,6-dihydro-6-methyl-2-oxo-1,3,4-thiadiazin-5-yl)-3,3-dimethyl-2-oxo-1H-indole-1-carbothioate

2,2,2-Trifluoroethyl 2,3-dihydro-5-(3,6-dihydro-6-methyl-2-oxo-1,3,4-thiadiazin-5-yl)-3,3-dimethyl-2-oxo-1H-indole-1-carboxylate

2-Methoxyethyl 2,3-dihydro-5-(3,6-dihydro-6-methyl-2-oxo-1,3,4-thiadiazin-5-yl)-3,3-dimethyl-2-oxo-1H-indole-1-carboxylate

2-(Methylthio)ethyl 2,3-dihydro-5-(3,6-dihydro-6-methyl- 2-oxo-1,3,4-thiadiazin-5-yl)-3,3-dimethyl-2-oxo-1H-indole-1-carboxylate

Ethyl 2,3-dihydro-5-(3,6-dihydro-6-methyl-2-oxo-1,3,4-thiadiazin-5-yl)-3,3-dimethyl-2-oxo-1H-indole-1-sulfinate

Cyclohexyl 2,3-dihydro-5-(3,6-dihydro-6-methyl-2-oxo-1,3,4-thiadiazin-5-yl)-3,3-dimethyl-2-oxo-1H-indole-1-carboxylate

2-Cyancethyl 2,3-dihydro-5-(3,6-dihydro-6-methyl-2-oxo-1,3,4-thiadiazin-5-yl)-3,3-dimethyl-2-oxo-1H-indole-1-carboxylate

Diethyl 2,3-dihydro-5-(3,6-dihydro-6-meth.yl-2-oxo-1,3,4-thiadiazin-5-yl)-3,3-dimethyl-2-oxo-1H-indole-1-phosphonate

2-Propenyl 2,3-dihydro-5-(3,6-dihydro-6-methyl-2-oxo-1,3,4-thiadiazin-5-yl)-3,3-dimethyl-2-oxo-1H-indole-1-carboxylate

3-[(4-Chlorophenyl)methyl]-1,3-dihydro-5-(3,6-dihydro-2-oxo-2H-1,3,4-thiadiazin-5-yl)-2H-indol-2-one

The compounds of the invention have two potential chiral centres at the $R_4/R_5$ and $R_1/R_2$ substituted positions, and can therefore exist in more than one stereoisomeric form. The invention extends to all such forms and to mixtures thereof, including all enantiomers, diastereomers, and racemates.

The pharmaceutically acceptable salts of the compounds of formula (I), (when the compound contains a salifiable group) include acid addition salts with conventional acids such as hydrochloric, hydrobromic, boric, phosphoric, sulphuric and pharmaceutically acceptable organic acids such as acetic, tartaric, maleic, citric, succinic, benzoic, ascorbic, methanesulphonic, α-keto-glutaric, α-glycerophosphoric, and glucose-1-phosphoric acids. Preferably the acid addition salt is a hydrochloride.

The compounds of the formula (I) and their pharmaceutically acceptable salts may also form solvates with pharmaceutically acceptable solvents, and such solvates are included within the expression 'a compound of formula (I)' used herein.

Salts of the compounds of the formula (I) which are not pharmaceutically acceptable may be useful as intermediates in the preparation of pharmaceutically acceptable salts of compounds of the formula (I) or the compounds of the formula (I) themselves, and as such form an aspect of the present invention.

The compounds of formula (I) in which A is sulphur may be prepared by treating a compound of formula (II)

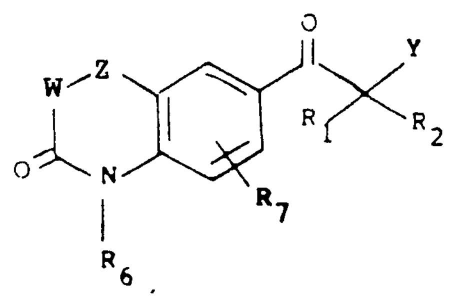

(II)

in which $R_1$, $R_2$, $R_6$, $R_7$, W and Z are as defined in formula (I), and
Y is a leaving group,
with a compound of formula (III)

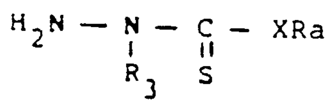

(III)

in which $R_3$ and X are as defined in formula (I), and $R_a$ is an alkyl group, preferably $C_{1-6}$ alkyl, for example methyl or ethyl, or an ammonium or alkali metal ion,
and thereafter optionally converting a compound wherein X is oxygen to a compound wherein X is sulphur by, for example, treatment with Lawessons reagent, and
thereafter if desired converting a compound of formula (I) thereby produced to a pharmaceutically acceptable salt thereof or to a further compound of formula I.

Y is preferably a halogen atom, an alkanesulphonyloxy group (such as methylsulphonyloxy) or arylsulphonyloxy (such as benzenesulphonyloxy or p-toluenesulphonyloxy). The reaction is preferably carried out in an organic solvent, for example ethanol, acetonitrile or dimethylformamide, at between room temperature and the boiling point of the solvent.

The compounds of formula (I) in which A is oxygen may be prepared by cyclising a compound of formula (IV):

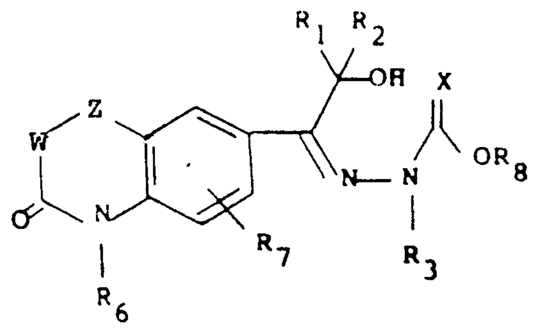

(IV)

in which $R_1$, $R_2$, $R_6$, $R_7$, X, W and Z are as defined in formula (I), and $R_8$ is an alkyl group, preferably $C_{1-6}$ alkyl such as methyl or ethyl. The cyclisation may be carried out in the presence of a base, such as sodium ethoxide, in a diluent or solvent, such as ethanol, at about ambient temperature.

The compounds of formula (I) in which A is -NH- may be prepared by treating a compound of formula (V):

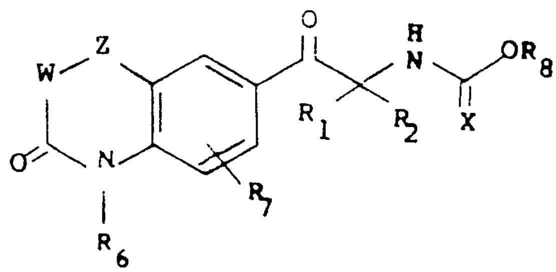

(V)

in which $R_1$, $R_2$, $R_6$, $R_7$, X, W and Z are as defined in formula (I) and $R_8$ is as defined in formula (IV), with a compound of formula (VI):

$H_2N$-NH-$R_3$ (VI)

in which $R_3$ is as defined in formula (I).

The reaction may be carried out in a diluent or solvent, such as ethanol, and suitably at a temperature up to the boiling point of the diluent or solvent.

The compounds of formula (I) may be converted into pharmaceutically acceptable salts in conventional manner by, for example, treatment with an appropriate acid.

Compounds of formula (I) may be converted to other compounds of formula (I) in accordance with known methods.

For example, some of the compounds of formula (I) may be prepared by treating the corresponding compound of formula (I) in which $R_6$ is hydrogen, with an acid chloride of formula $R_6$-Hal, where Hal is halogen, preferably chlorine.

Also, compounds of formula (I) in which $R_4$ and $R_5$ together form a heterocyclic ring containing two sulphur atoms may be prepared by treating the corresponding compound of formula (I) in which $R_4$ and $R_5$ together form an oxo group, with an alkanedithiol.

The compounds of formula (II) may themselves be prepared by treating a compound of formula (VII):

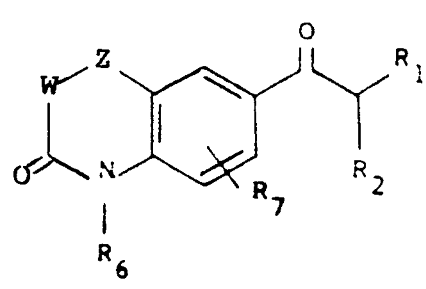

(VII)

in which $R_1$, $R_2$, $R_6$, $R_7$, W and Z are as defined in formula (I), with a compound containing the leaving group Y or, when Y is halogen, with the halogen itself.

When Y is halogen, it is preferably bromine or chlorine.

The compounds of formula (VII) may be prepared in several ways, as summarised in the following Schemes A, B and C.

<u>Scheme A</u>

This Scheme may be used for preparing compounds of formula (VII) in which $R_4$ or $R_5$ is $C_{1-3}$ alkylthio and n is zero, and is exemplified by $R_4$ as methyl, and $R_5$ as methylthio.

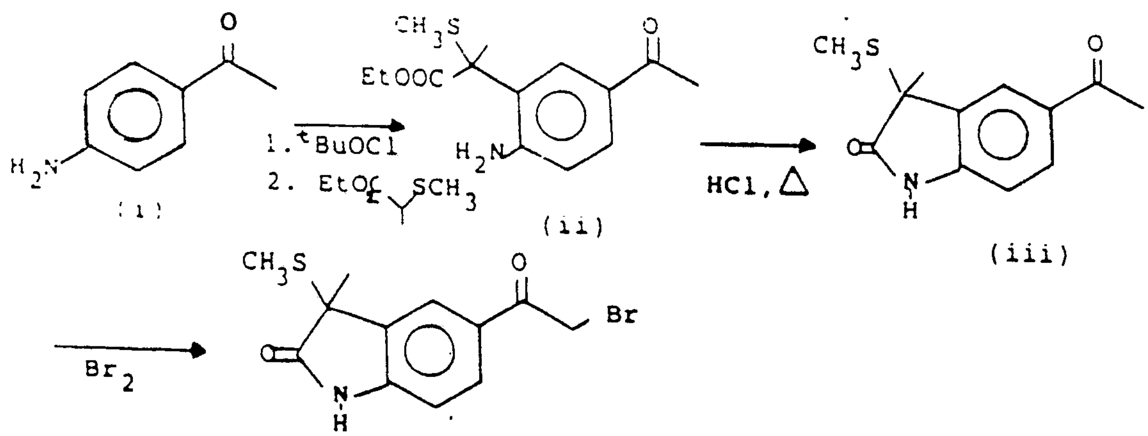


A reaction of this type is described in J. Am. Chem. Soc., <u>96</u>, 5508, 1974 (Gassman and Bergen).

<u>Scheme B</u>

This scheme is suitable for preparing compounds in which one or both of $R_4$ and $R_5$ are $C_{1-3}$ alkyl, or $R_4$ and $R_5$ together form a ring as defined in formula (II), and n is zero, and is exemplified by $R_4$ and $R_5$ as methyl.

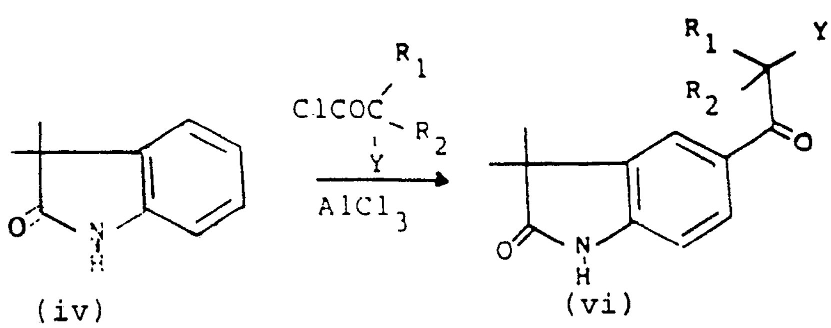


<u>Scheme C</u>

This is a variant of Scheme B, in which an additional step is present

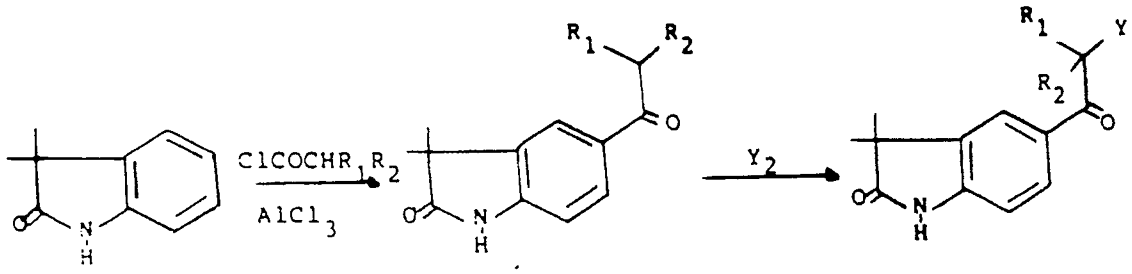


(iv) (v)

In the above schemes, the starting compounds of formulae (i) and (iv) are known compounds; compound (iv) is disclosed in Monatsh., 58, 369-398, 1931 and spiro analogues thereof (when $R_4$ and $R_5$ form a ring) are described in Beilstein 21 II 269-268.

The compounds of formulae (III) are also known compounds, or are preparable in analogous manner to the known compounds of formula (III).

Compounds of formula (IV) may be prepared by treating a compound of formula (VIII):

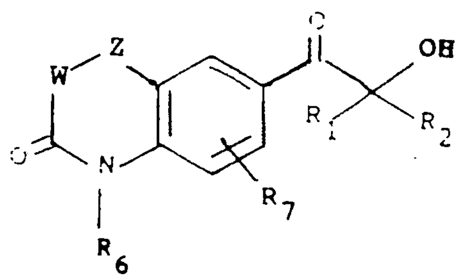

(VIII)

in which $R_1$, $R_2$, $R_6$, $R_7$, W and Z are as defined in formula (I), with an alkyl carbazate of formula (IX):

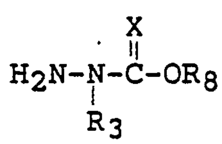

(IX)

in which X, $R_3$ and $R_8$ are as defined in formula (IV).

Compounds of formula (VIII) may themselves be prepared by hydrolysing a compound of formula (II).

Compounds of formula (V) may be prepared by treating a compound of formula (X):

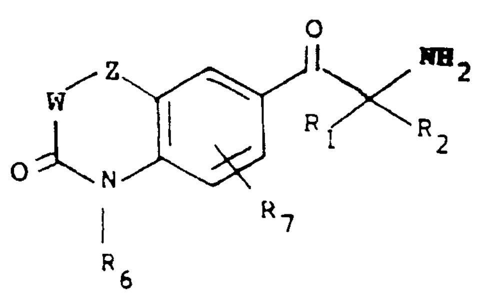

(X)

in which $R_1$, $R_2$, $R_6$, $R_7$, W and Z are as defined in formula (I),
with a compound of formula (XI):

Cl- C -$OR_8$ (XI)
X

in which X and $R_8$ are as defined in formula (IV).

Compounds of formula (X) may themselves be prepared by treating a compound of formula (II) with sodium azide, and reducing the thus formed azide with hydrogen in the presence of a palladium/charcoal

catalyst.

Compounds of formula (VI), (IX) and (XI) are known compounds, or can be prepared from known compounds by known methods.

A number of the intermediate compounds of formulae (II), (IV), (V), (VII), (VIII) and (X) are novel and form a further aspect of the invention.

In order to utilise the potential cardiotonic and anti-asthmatic activities of the compounds of formula (I) the invention also provides a pharmaceutical composition comprising a compound of formula (I), or a pharmaceutically acceptable salt thereof, and a pharmaceutically acceptable carrier.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, in particular shaped oral compositions, since they are more convenient for general use.

Tablets and capsules for oral administration are usually presented in a unit dose, and contain conventional excipients such as binding agents, fillers, diluents, tableting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate.

Suitable lubricants include, for example, magnesium stearate. Suitable pharmaceutically acceptable wetting agents include sodium lauryl sulphate.

These solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

For parenteral administration, fluid unit dose forms are prepared containing a compound of the present invention and a sterile vehicle. The compound, depending on the vehicle and the concentration, can be either suspended or dissolved.

Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the compound of the invention.

In addition such compositions may contain further active agents such as vasodilator agents and diuretics.

As is common practice, the compositions will usually be accompanied by written or printed directions for use in the medical treatment concerned.

The invention further provides a method of treatment or prophylaxis of heart disease or asthmatic conditions in mammals, such as humans, which comprises the administration of an effective amount of a compound of formula (I), or a pharmaceutically acceptable salt thereof, to the sufferer.

An amount effective to treat the disorders hereinbefore described depends on the relative efficacies of the compounds of the formula (I), the nature and severity of the disorders being treated and the weight of the mammal. However, a unit dose will normally contain 1 to 100 mg for example 2 to 50 mg, of the compound of the invention. Unit doses will normally be administered once or more than once a day, for example 2,3,4,5 or 6 times a day, more usually 2 to 4 times a day, such that the total daily dose is normally in the range, for a 70 kg adult of 1.0 to 2500 mg, more usually 50 to 2000 mg, for example 10 to 75mg, that is in the range of approximately 0.002 to 35 mg/kg/day, more usually 1 to 30 mg/kg/day, for example 0.15

to 1 mg/kg/day.

In an additional aspect of the invention there is provided a compound of formula (I) or a pharmaceutically acceptable salt thereof, for use as an active therapeutic substance, and in particular for the treatment of heart disease or asthmatic conditions.

Furthermore, the invention provides the use of a compound of formula (I) or a pharmaceutically acceptable salt thereof in the manufacture of a medicament for the treatment of heart disease or asthmatic conditions.

The following examples illustrate the compounds of the invention and the following descriptions illustrate intermediates thereto. The following pharmacological data illustrate the activity of the compounds of the invention.

Description 1

1,3-Dihydro-3,3-dimethyl-5-(1-oxopropyl)-2H-indol-2-one

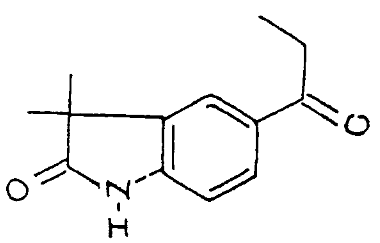

(D1)

37.7g aluminium chloride and 6.5 ml DMF were mixed and heated with stirring at 70°C for 30 minutes. After cooling to 60°C 5g (31 mmol) 1,3-dihydro-3,3-dimethyl-2H-indol-2-one (Monatsh. Chem., 18, 533, 1897) and 2.87g (31 mmol) propionyl chloride were added and stirred at 60°C for 2.5 hours. The mixture was poured onto 300g ice. The precipitate was filtered, washed with water and triturated with petrol-ether affording 6.36g of the desired compound (94%).

m.p. = 197°C

IR (KBr) $\nu$ = 3,200; 1,720; 1,680; 1,200 $cm^{-1}$.

NMR ($CDCl_3$) $\delta$ = 1.24 ppm (t,3H,J=7.3Hz,$CH_3$-$CH_2$); 1.44 (s,6H,2 x $CH_3$-C); 2.99 (q,2H,J=7.3Hz,$CH_2$-$CH_3$); 7.01 (d,1H,J=8.7Hz,Ar); 7.85-7.95 (m,2H,Ar); 9.29 (s,1H,exch.$D_2$0,NH).

Description 2

2,3-Dihydro-3,3-dimethyl-2-oxo-5-(1-oxopropyl)-N-phenyl-1H-indole-1-carboxamide

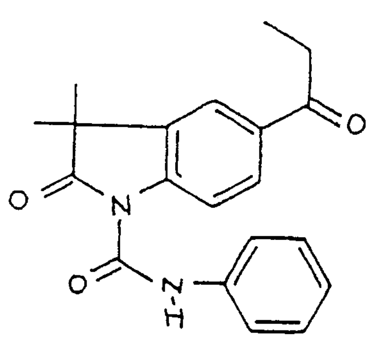

(D2)

2.5g (11.5mmol) 1,3-dihydro-3,3-dimethyl-5-(1-oxopropyl)-2H-indol-2-one (Description 1), 4.1g (34mmol) phenyl isocyanate and 25ml toluene were refluxed for 2 hrs. After cooling the precipitate was filtered,

washed with ether and dried, yielding 3.4g (92%) of the title compound.
m.p. = 174 ° C.
IR (KBr) ν = 3,200; 1,740; 1,680; 1,600; 1,550 ; 1,200 $cm^{-1}$.

Description 3

5-(2-bromo-1-oxo-1-propyl)-2,3-dihydro-3,3-dimethyl-2-oxo-N-phenyl-1H-indole-1-carboxamide

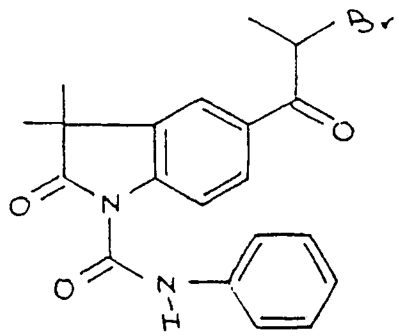

(D3)

1.6g (10mmol) bromine in 6ml chloroform were added dropwise to a mixture of 3.4g (10mmol) 2,3-dihydro-3,3-dimethyl-2-oxo-5-(1-oxopropyl)-N-phenyl-1H-indole-1-carboxamide (Description 2), a few drops of dioxane and 60ml chloroform. Stirring was maintained for 30 minutes after the end of the addition. The reaction mixture was washed with water, diluted $NaHCO_3$ again with water, dried over $MgSO_4$ and evapored to dryness. The residue was chromatographed on silica and eluted with chloroform/hexane : 8/2 yielding 3.5g (84%) of the desired compound.
m.p. = 176 ° C.
IR (KBr) ν = 3,200; 1,740; 1,675; 1,605; 1,555; 1,220 $cm^{-1}$
NMR ($CDCl_3$) δ = 1.54 ppm (s,6H,$CH_3$-C); 1.93 (d,3H,J=6.6Hz,$CH_3$-CH); 5.31 (q,1H,J=6.6Hz,CH-$CH_3$); 7.14-7.61 (3m,1H,2H,2H,Ph); 7.97 (d,1H,J′=1.9Hz,Ar); 8.03 (dd,1H,J=8.6Hz,J′=1.9Hz,Ar); 8.46 (d,1H,J=8.6Hz,Ar); 10.66 (s,1H,exch.$D_2O$,NH).

Description 4

Ethyl 2,3-dihydro-3,3-dimethyl-2-oxo-1H-indole-1-acetate

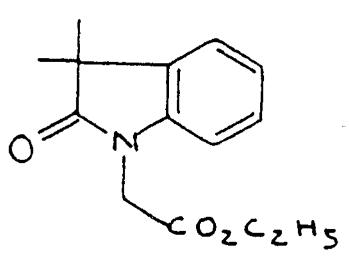

(D4)

To a solution of 1.6g (10mmol) 1,3-dihydro-3,3-dimethyl-2H-indol-2-one in 50ml DMF, 0.44g (10mmol) of NaH (55% suspension in oil) were added. When gas evolution had ceased, the mixture was heated at 40 ° C for 30 minutes and then cooled below 0 ° C with an ice-salt mixture. Then, 1.82g (11mmol) ethyl 2-bromo acetate was added dropwise. 15 minutes after the end of the addition, the solvent was removed

under reduced pressure. The residue was taken up in dichloromethane, washed with water, dried over $MgSO_4$ and concentrated to dryness affording 2.02 (82%) of an oil used directly in the next step.
IR (film) $\nu$ = 3,000; 1,750; 1,720; 1,620; 1,200 $cm^{-1}$.
NMR ($CDCl_3$) $\delta$ = 1.25 ppm (t,3H,J=7.2Hz,$CH_3$-$CH_2$); 1.42 (s,6H,$CH_3$-C); 4.20 (q,2H,J=7.2Hz,$CH_2$-$CH_3$); 4.47 (s,2H,N-$CH_2$); 6.71-6.75 (m,1H,Ar); 7.04-7.11 (m,1H,Ar) ; 7.19-7.29 (m,2H,Ar).

Description 5

Ethyl-5-(2-bromo-1-oxo-1-propyl)-2,3-dihydro-3,3-dimethyl-2-oxo-1H-indole-1-acetate

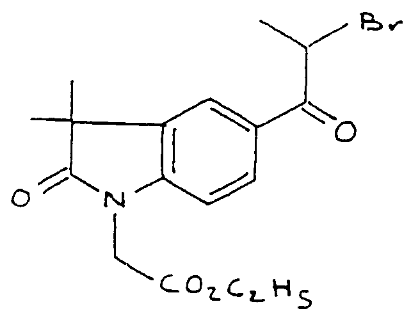

(D5)

10.6g (79mmol) anhydrous aluminum chloride and 2.03ml DMF were mixed and heated with stirring at 70°C for 30 minutes. After cooling at room temperature 1.97g (79mmol) ethyl 2,3-dihydro-3,3-dimethyl-2-oxo-1H-indole-1-acetate (Description 4) and 2-bromo-propionyl chloride were added and stirred 1hr at room temperature and over night at 40°C. Then, the mixture was poured onto 150g crushed ice. After a few minutes the precipitate was filtered, washed with 50ml ethanol and dried affording 3g (66%) of the desired compound.
IR (KBr) $\nu$ = 2,950; 1,740; 1,725; 1,680; 1,610; 1,220 $cm^{-1}$
NMR (DMSO-$d_6$) $\delta$ = 1.20 ppm (t,3H,J=7.1Hz,$CH_3$-$CH_2$); 1.35 (s,6H,$CH_3$-C); 1.78 (d,3H,J=6.4Hz,$CH_3$-CH); 4.15 (q,2H,J=7.1Hz,$CH_2$-$CH_3$); 4.64 (s,2H,N-$CH_2$); 5.85 (q,1H, J=6.4Hz,CH-$CH_3$); 7.21 (d,1H,J=8.3Hz,Ar) ; 8.02-8.08 (m,2H,Ar).

Description 6

α-(Acetyloxy)-N[4-(2-chloro-1-oxo-1-propyl)phenyl]benzene acetamide

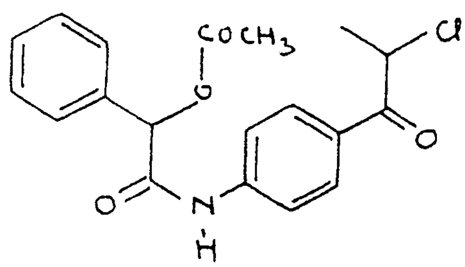

(D6)

10.6g (50mmol) O-acetylmandelyl chloride in 20ml chloroform were added slowly, at room temperature, to a mixture of 9.2g (50mmol), 1-[(4-amino)phenyl]-2-chloro propanone, 100ml chloroform and 5g triethylamine and then heated 0.5hr to reflux. After cooling the reaction mixture was washed with water, sodium carbonate, and again with water. The organic phase was dried over $MgSO_4$ and concentrated. The oily residue (12g) was used directly in the next step.

IR (film) $\nu$ = 3,350; 1,730; 1,690; 1,600; 1,230 $cm^{-1}$.

Description 7

5-(2-Chloro-1-oxo-1-propyl)-1,3-dihydro-3-phenyl-2H-indol-2-one

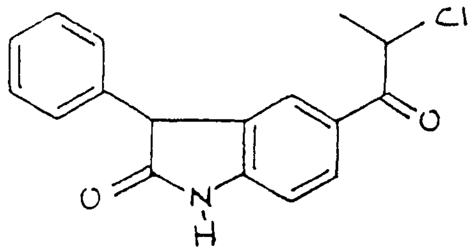

(D7)

12g (33mmol) of the oil of Description 6 were heated at 60° C for 1.5 hr in 100ml concentrated sulphuric acid. The reaction mixture was poured onto 500g crushed ice, the precipitate was extracted twice with 125ml ethyl acetate, the organic phase washed twice with water, dried and concentrated. The residue was purified by column chromatography on silica (eluent : hexane/ethylacetate : 1/1) giving 4g of the desired compound.

NMR ($CDCl_3$) : $\delta$ = 1.71 ppm (d,3H,J=6.6Hz,$CH_3$-CH) ; 4.70 (s,1H,øCH) ; 5.16 (m,1H,CH-$CH_3$) ; 7.03 (d,1H,J=8.2Hz, Ar) ; 7.19-7.41 (m,5H,Ar) ; 7.82 (d,1H,J=6.1Hz,Ar) ; 7.99 (d,1H,J=8.2Hz,Ar) ; 9.17 (s,1H,exch.$D_2O$,NH).

Description 8

1,3-Dihydro-3,3-dimethyl-5-(1-oxopropyl)-1-(phenylmethyl)-2H-indol-2-one

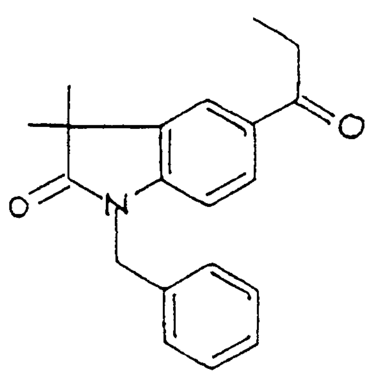

(D8)

3g (13.8mmol) 1,3-dihydro-3,3-dimethyl-5-(1-oxopropyl)-2H-indol-2-one (Description 1) were dissolved in 30ml DMF and the solution cooled to 0° C. Then one equivalent of NaH (55% in oil) was added and the mixture brought to room temperature. 1.75g (13.8mmol) benzylchloride, dissolved in 10ml DMF, were added. After 0.5 hr, the mixture was heated at 60° C for 1 hr.

The solvent was concentrated under reduced pressure, the residue taken up in 100ml ethylacetate and washed twice with 100ml of water. The organic solution was dried over $MgSO_4$ and concentrated to dryness. Trituration with isopropyl ether afforded 4g (94%) of the desired compound.

m.p. = 85° C.

NMR ($CDCl_3$) : $\delta$ = 1.21 ppm (t,3H,J=7.3Hz,$CH_3$-$CH_2$) ; 1.47 (s,6H,$CH_3$-C) ; 2.94 (q,2H,J =7.3Hz,$CH_2$-$CH_3$) ; 4.95 (s,2H,N-$CH_2$) ; 6.76 (d,1H,J=8.2Hz,Ar) ; 7.26-7.32 (m.5H,Ar);7.81 (dd,1H,J=8.2Hz,J′ =1.7Hz,Ar) ; 7.87 (d.1H,J′ =1.7Hz,Ar).

Description 9

5-(2-bromo-1-oxo-1-propyl)-1,3-dihydro-3,3-dimethyl-1-(phenylmethyl)-2H-indol-2-one

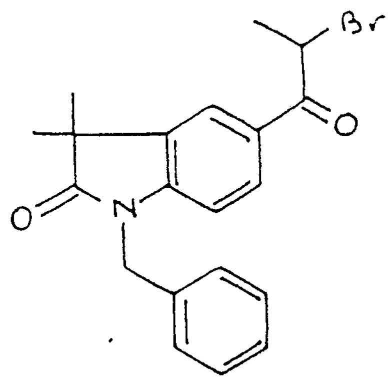

(D9)

2.08g (13mmol) bromine in 20ml chloroform were added dropwise, at 60 °C, to a mixture of 4g (13mmol)1,3-dihydro-3,3-dimethyl-5-(1-oxopropyl)-1-(phenylmethyl)-2H-indol-2-one (Description 8). The solvent was then concentrated to dryness, the residue taken up in 100ml ethyl acetate, washed twice with 100ml water. The organic phase was dried over $MgSO_4$ and concentrated to dryness. The residue was purified by column chromatography on silica (eluent : hexane/ethylacetate : 2/1) giving 2.86g (56%) of the desired compound.
m.p. = 119 °C.
NMR ($CDCl_3$) : $\delta$ = 1.48 ppm (s,6H,$CH_3$-C) ; 1.88 (d,3H,J=6.6Hz,$CH_3$-CH) ; 4.95 (s,2H,N-$CH_2$) ; 5.24 (q,1H,J=6,6Hz,CH-$CH_3$) ; 6.79 (d,1H,J=8.2Hz,Ar) ; 7.24-7.38 (m,5H,Ar) ; 7.87-7.93 (m,2H,Ar)

Description 10

(2-methyl) propanoic acid 2,2-diphenyl hydrazide

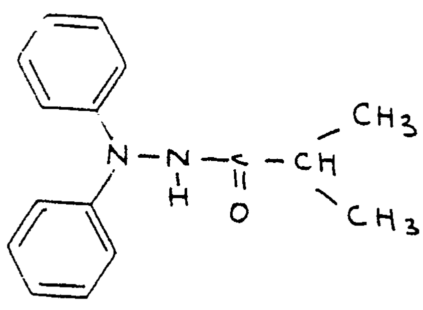

(D10)

To a mixture of 18.4g (0.1mol) 1,1-diphenylhydrazine, 15.8g (0.2mol) pyridine and 100ml DMF, 10.6g (0.1mol) isobutyryl chloride in 100ml DMF were added. The mixture was stirred 2hrs at room temperature, then the solvent removed under reduce pressure. The residue was dissolved in 100ml ethyl acetate, the organic phase washed twice with water, dried over $MgSO_4$ and concentrated to dryness. The residue was crystallised by trituration with isopropyl ether affording 25g (90%) of the desired compound used directly in the next step.

Description 11

1,3-Dihydro-3,3-dimethyl-1-phenyl-2H-indol-2-one

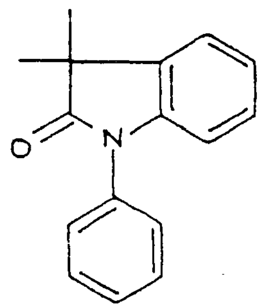

(D11)

A mixture of 7g (25mmol) (2-methyl)propanoic acid 2,2-diphenyl hydrazine (Description 10), 2.1g (50mmol) calcium hydride and 25ml 1,2,3,4-tetrahydronaphthalene were refluxed for 3hrs. The solid was filtered off and the solvent concentrated under reduced pressure. The residue was acidified with 100ml 1N HCl and then extracted twice with 100ml ethyl acetate. The organic phase was washed with water dried over $MgSO_4$ and concentrated to dryness. Trituration of the residue with isopropyl ether afforded 4.2g (71%) of the desired compound.
m.p. = 75° C.
NMR ($CDCl_3$) : $\delta$ = 1.49 (s,6H,$CH_3$-C-$CH_3$) ; 6.83 (d,1H,Ar) ; 7.0-7.5 (m,8H,Ar).

Description 12

5-(2-bromo-1-oxo-1-propyl)-1,3-dihydro-3,3-dimethyl-1-phenyl-2H-indol-2-one

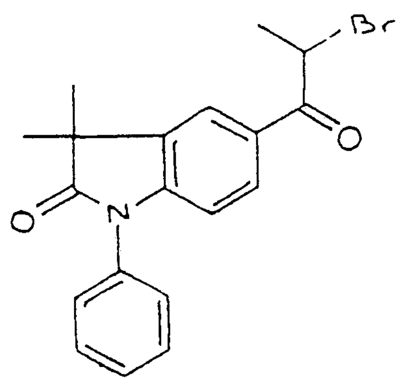

(D12)

3ml DMF were added dropwise to 23g anhydrous aluminium chloride and the exothermic reaction was then allowed to cool to room temperature. Then, 4.2g (17.7mmol) of 1,3-dihydro-3,3-dimethyl-1-phenyl-2H-indol-2-one (Description 11) and 3.82g (18mmol) of 2-bromo propionyl chloride were added and stirred at room temperature for 1 hour. The reaction mixture was poured on 100g crushed ice containing 5ml 10N hydrochloric acid. The mixture was extracted twice with 50ml ethyl acetate. The organic phase was washed with water until neutral, dried over $MgSO_4$ and concentrated to dryness.
Purification by column chromatography on silica (elution by hexane/ethyl acetate : 1/1) afforded 3g (46%) of the desired compound.
m.p. = 140° C.
IR (KBr) $\nu$ = 1,730 ; 1,680 ; 1,615 ; 1,500 ; 1,220 $cm^{-1}$.
NMR ($CDCl_3$) : $\delta$ = 1.53 (s,6H,$CH_3$-C-$CH_3$) ; 1.91 (d,J=7Hz,3H,$CH_3$-CH) ; 5.29 (q,J=7Hz,1H,CH-$CH_3$) ; 6.91 (d,1H,Ar) ; ≈ 7.5 (m,5H,Ar) ; 7.90 (dd,1H,Ar) ; 7.99 (d,1H,Ar).

Description 13

1,3-Dihydro-3,3-dimethyl-1-(2-phenylethyl)-2H-indol-2-one

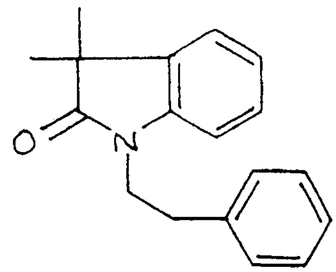

(D13)

A solution of 6.44g (40mmol) 1,3-dihydro-3,3-dimethyl-2H-indol-2-one (Monatsh.Chem.,18,533,1897) in 100ml dry dimethylformamide is added dropwise to 1.74g (40mmol) sodium hydride (55% suspension in oil) at 0°C. When gas evolution had ceased, 7.4g (40mmol) 2-phenyl ethyl bromide were added dropwise and the resulting suspension was stirred 1 hour at room temperature, then concentrated to dryness. The residue was poured on ice, extracted twice with 200ml ethyl acetate. The organic phases were washed with water and dried over $MgSO_4$. The compound was purified by column chromatography on silica yielding 5.58g (56%) of an oil.
IR (KBr) : $\nu$ = 2,925 ; 2,975 ; 1,715 ; 1,618 ; 1,490 ; 1,360 ; 1,165 $cm^{-1}$.
NMR ($CDCl_3$) : $\delta$ = 1.31 (s,6H,2 x $CH_3$) ; 2.97 and 3.93 (2 x t,2 x 2H,J=8Hz,Ar$CH_2CH_2$N) ; 6.8-7.3 (m,9H,Ar).

Description 14

5-(2-Bromo-1-oxo-1-propyl)-1,3-dihydro-3,3-dimethyl-1-(2-phenylethyl)-2H-indol-2-one

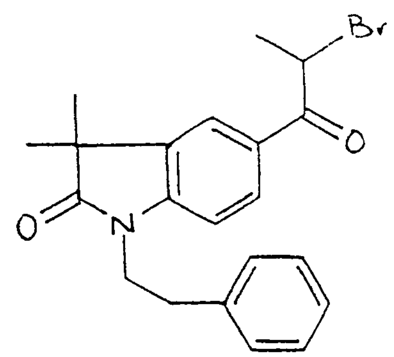

(D14)

Starting from 1,3-dihydro-3,3-dimethyl-1-(2-phenylethyl)-2H-indol-2-one (Description 13) and following the procedure of Description 12, afforded the desired compound as an oil which was used in the next step without further purification.
IR (KBr) : $\nu$ = 2,970 ; 2,920 ; 1,710 ; 1,690 ; 1,615 ; 1,492 ; 1,385 ; 1,360 ; 1,240 ; 1,165 $cm^{-1}$.
NMR ($CDCl_3$) : $\delta$ = 1.30 (s,6H,2 x $CH_3$) ; 1.88 (d,J=6Hz,3H,$CH_3$CHBr) ; 3.06 and 3.99 (2 x t, J=7Hz,2 x 2H, Ar$CH_2CH_2$N); 5.25 (q,J=6Hz,1H,CHBr) ; 6.8-8.0 (m,8H,Ar).

Description 15

1-Cyclohexylmethyl-1,3-dihydro-3,3-dimethyl-5-(1-oxopropyl)-2H-indol-2-one

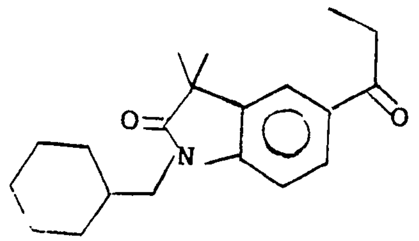

(D15)

10.6g (49mmol) 1,3-dihydro-3,3-dimethyl-5-(1-oxopropyl)-2H-indol-2-one (Description 1) were dissolved in 100ml dry DMF. To the cooled solution (0° C), 2.14g (49 mmol) sodium hydride (55% suspension in oil) were added over a 30 minute period. The solution was stirred for 30 minutes at room temperature, cooled to 10° C and 8.67g (49 mmol) cyclohexylmethyl bromide were added over a 60 minute period. The mixture was stirred for 1 hour at room temperature and another hour at 50° C. The mixture was concentrated to dryness, taken up in 200ml ethyl acetate, washed with water (2x100ml) and dried over magnesium sulphate. After concentration the residue was triturated with isopropyl ether to give the desired compound. Yield 51%.
m.p.: 105° C
IR (KBr): $\nu$ = 2920; 2850; 1715; 1680; 1622; 1385 $cm^{-1}$.
NMR ($CDCl_3$): $\delta$ = 0.9-2.0 (m, 20H, cyclohexane (11H) and including at 1.24 ppm, J = 7Hz, 3H, $CH_3$ $CH_2$ and s at 1.40 ppm, 6H, $(CH_3)_2C$); 2.98 (q, J = 7Hz, 2H, $COCH_2$); 3.56 (d, J = 7Hz, 2H, $CH_2N$); 6.88 (d, J = 8Hz, 1H, Ar); 7.86 (d, J' ≃ 2Hz, 1H, Ar); 7.92 (dd, J = 8Hz, J' ≃ 2Hz, 1H, Ar).

Description 16

5-(2-Bromo-1-oxopropyl)-1-cyclohexylmethyl-1,3-dihydro-3,3-dimethyl-2H-indol-2-one

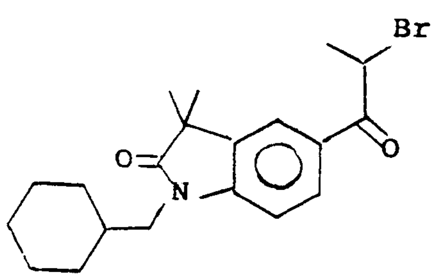

(D16)

6.28g (20 mmol) 1-cyclohexylmethyl-1,3-dihydro-3,3-dimethyl-5-(1-oxopropyl)-2H-indol-2-one (Description 15) were dissolved in 150ml chloroform and 30ml dioxane under argon. 3.18 (20 mmol) bromine in 20ml chloroform were added dropwise at 60° C over a 30 minute period. The resulting colourless solution was then poured on to 500ml water, decanted, washed with water (2x250ml) and dried over magnesium sulphate. After distillation of the solvent, the oily residue was crystallized from isopropyl ether and heptane (1:1). The compound was used in the next step without further purification. Yield 89%.
m.p.: 95° C.
IR (KBr): $\nu$ = 2920; 2850; 1715; 1675; 1610; 1390; 1360 $cm^{-1}$.
NMR ($CDCl_3$): $\delta$ = 0.9-2.0 (m, 20H, cyclohexane (11H) and including s at 1.41ppm, 6H, $(CH_3)_2C$, and d at 1.91 ppm, J = 7Hz, 3H, $CH_3$ CH); 3.57 (d, J = 7Hz, 2H, N-$CH_2$); 5.28 (q, J = 7Hz, 1H, $CH_3CH$); 6.91 (d, J = 8Hz, 1H, Ar); 7.90 (d, J ≃ 2Hz, 1H, Ar); 7.98 (dd, J = 8Hz, J' ≃ 2Hz 1H, Ar).

Example 1

1′-[(4-Aminophenyl)carbonyl]-5′-(3,6-dihydro-6-methyl-2-oxo-2H-1,3,4-thiadiazin-5-yl)-spiro[cyclopentane-1,3′-[3H]-indol]-2′(1′H)-one, hydrochloride

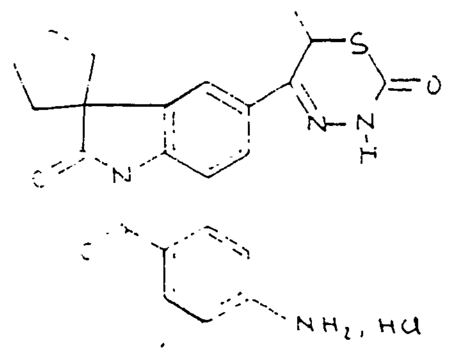

(E1)

(A) A mixture of 4.2g (9mmol) 5′-(3,6-dihydro-6-methyl-2-oxo-2H-1,3,4-thiadiazin-5-yl)-1′-[(4-nitrophenyl)carbonyl]-spiro[cyclopentane-1,3′-[3H]-indol]-2′(1′H)-one and 10.2g (226 mmol) tin II chloride dihydrate in 100ml ethanol was stirred for 24 hours at room temperature. The solution was concentrated, suspended in 100ml ethyl acetate, and 100ml of a saturated solution of sodium hydrogen carbonate were added. The mixture was filtered, decanted and the organic layer was washed with water, dried over magnesium sulphate and concentrated to≈10ml. The crystals were filtered, washed with ethyl acetate/hexane : 1/1 and dried under vacuum affording 2.2g (56%) of the desired compound.
m.p. = 251-2° C.
IR (KBr)ν = 3,370 ; 3,200 ; 1,750 ; 1,660 ; 1,625 ; 1,605; 1,280 $cm^{-1}$.
NMR (DMSO-$d_6$) : δ = 1.49 ppm (d,3H,J=7.0Hz,$CH_3$-CH) ; 1.98-2.18 (m,8H,cyclopentyl) ; 3.10-3.85 (broad, exch. $D_2O$,$NH_2$.HCl) ; 4.81 (q,1H,J=7.0Hz,CH-$CH_3$) ; 6.58 (d,2H,J=8.7Hz,Ar) ; 7.33 (d,1H,J=8.4Hz,Ar) ; 7.53 (d,2H, J=8.7Hz,Ar) ; 7.75 (d,1H,J=8.4Hz,Ar) ; 7.82 (s,1H,Ar) ; 11.67 (s,1H,exch.$D_2O$,NH).

(B) 5′-(3,6-Dihydro-6-methyl-2-oxo-2H-1,3,4-thiadiazin-5-yl)-1′[(4-nitrophenyl)carbonyl]-spiro[cyclopentane-1,3′-[3H]indol]-2′(1′H)-one

0.45g (0.01mmol) NaH 55% (suspension in oil) were added to an ice cooled solution of 3.15g (0.01mmol) 1′,3′-dihydro-5′-(3,6-dihydro-6-methyl-2-oxo-2H-1,3,4-thiadiazin-5-yl)-spiro [cyclopentane-1,3′-2H-indol]-2(1′H)-one in 50ml DMF. After dissolution of the hydride, 1.9g (0.01mmol) 4-nitrobenzoylchloride were added progressively. Stirring was continued for 2 hours at room temperature. The mixture was poured on water and extracted with ethyl acetate. The organic phase was washed with brine, then with water, dried over magnesium sulphate and concentrated. Purification by chromatography on silica (eluent hexane/ethylacetate : 1/1) afforded 1.9g of the desired compound (41%).
m.p. = 231-3° C.
IR (KBr): ν = 3,250; 2,950; 1,765; 1,680; 1,660; 1,619; 1,530; 1,345; 1,310 $cm^{-1}$.
NMR ($CDCl_3$): δ 1.71 ppm (d, J=7.3Hz, 3H, $CH_3$ CH); 1.9-2.4 (m, 8H, cyclopentane); 4.32 (q, 7.3Hz, 1H, $CH_3$CH); 7.63 (dd, 1H, J=1.9Hz, J′=8.6Hz, Ar); 7.80 (d, J=8.7Hz, 2H, $ArNO_2$); 7.79 (d, 1H, J=1.9Hz, Ar); 8.07 (d, J′=8.6Hz, 1H, Ar); 8.33 (d, J=8.7Hz 2H, $ArNO_2$); 9.01 (s, 1H, exch. $D_2O$, NH).

(C) 1′,3′-Dihydro-5′-(3,6-dihydro-6-methyl-2-oxo-H-1,3,4-thiadiazin-5-yl)spiro[cyclopentane-1,3′-2H-indol]-2′-one

174g (1.3mol) aluminium chloride and 30ml DMF were mixed and heated with stirring at 70 C for 30 minutes. After cooling to 40° C, 24.4g (0.13mmol) spiro[cyclopentane-1,3′-[3H]-indol]-2′-one and 28.2g (0.13mmol) 2-bromopropionylbromide were added and stirred at 70° C for 1 hour. The mixture was poured onto 1000g ice and 85ml 10N HCl, then extracted with 3x500ml ethylacetate. The organic layer was washed with water, dried over $MgSO_4$ and evaporated to dryness. The crystalls were collected and washed with a

mixture of hexane/ethylacetate 1/1 giving 26g (62%) of the desired compound. m.p. = 190° C.

These 26g (0.08mol) were dissolved, with 8.6g (0.08mol) 0-methyl thiocarbazate, in 200ml acetonitrile and refluxed for 2.5 hours. During cooling the compound crystallized. The filtered precipitate was washed with acetonitrile giving 13.7g of the desired compound (Yield: 54%: overall yield 33.6%)
m.p. = 275° C.
IR (KBr) $\nu$ = 3,200; 1,705; 1.690; 1,485; 1,240 $cm^{-1}$.
NMR (DMSO-$d_6$) $\delta$ = 1.61 ppm (d, 3H, J=7.2Hz, $CH_3$); 2.00 (m, 8H, cyclopentane); 4.36 (q, 1H, J=7.2Hz, CH); 7.00 (d, 1H, J=8Hz, Ar); 7.49 (d, 1H, J=8Hz, Ar); 7.65 (s, 1H, Ar); 10.15 (s, 1H, exch. $D_2O$, NH); 11.07 (s, 1H, exch. $D_2O$, NH).

Example 2

2,3-Dihydro-5-(3,6-dihydro-6-methyl-2-oxo-2H-1,3,4-thiadiazin-5-yl)-3,3-dimethyl-2-oxo-N-phenyl-1H-indole-1-carboxamide

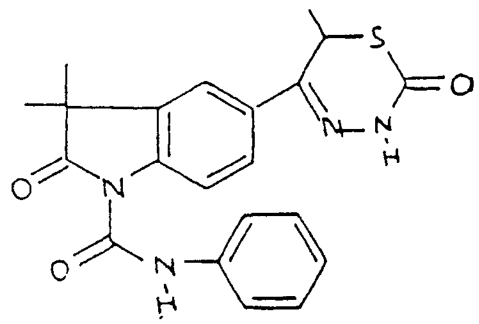

(E2)

3.5g (8.4mmole) 5-(2-bromo-1-oxo-1-propyl)-2,3-dihydro-3,3-dimethyl-2-oxo-N-phenyl-1H-indole-1-carboxamide (Description 3), 0.98g (9.2mmol) O-methyl thiocarbazate and 45ml acetonitrile, were refluxed for 4 hours. After cooling the precipitate was isolated by filtraton, washed with acetonitrile and dried to give 1.35g (39%) of the desired compound.
m.p. = 212° C.
IR (KBr)$\nu$ = 3,200 ; 1,740 ; 1,630 ; 1,600 ; 1,550 ; 1,240 $cm^{-1}$.
NMR (DMSO-$d_6$) : $\delta$ = 1.47-1.53 ppm (m,9H,$CH_3$-C et $CH_3$-CH) ; 4.78 (q,1H,J=7.1Hz,CH-$CH_3$) ; 7.13 -8.13 (6m,1H,2H,2H,1H,1H,1H,Ar) ; 10.57 (s,1H,exch.$D_2$0,NH) ; 11.71 (s,1H,exch.$D_2$0,NH).

Example 3

Ethyl 2,3-dihydro-5-(3,6-dihydro-6-methyl-2-oxo-2H-1,3,4-thiadiazin-5-yl)-3,3-dimethyl-2-oxo-N-1H-indole-1-acetate

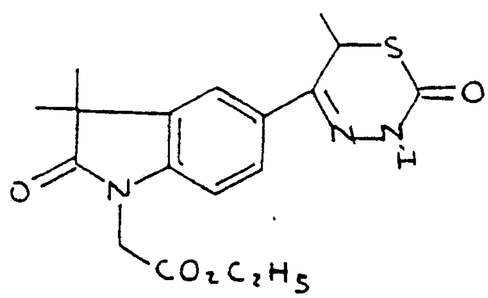

(E3)

1.47g (3.8mmol) ethyl 5-(2-bromo-1-oxo-1-propyl)-2,3-dihydro-3,3-dimethyl-2-oxo-1H-indole-1-acetate

(Description 5), 0.41g (3.8mmol) 0-methyl thiocarbazate and 20ml acetonitrile were refluxed for 16hrs. After evaporation of the solvent the residue was purified by column chromatography on silica (eluent : dichloromethane ethyl acetate : 8/2). The major fraction was triturated in ether to yield a white solid.
m.p. : 191 °C.
IR (KBr)$\nu$ = 3,200 ; 1,750 ; 1,720 ; 1,640 ; 1,620 ; 1,210 $cm^{-1}$.
NMR (DMSO-$d_6$) : $\delta$ = 1.16 ppm (t,3H,J = 7.1Hz,$CH_3$-$CH_2$) ; 1.33 (s,6H,$CH_3$-C) ; 1.49 (d,3H,J = 7.0Hz,$CH_3$-CH) ; 4.14 (q,2H,J = 7.1Hz,$CH_2$-$CH_3$) ; 4.61 (s,2H,N-$CH_2$) ; 4.56 (q,1H,J = 7.0Hz,CH-$CH_3$) ; 7.14 (d,1H,J = 8.3Hz,Ar) ; 7.71 (dd,1H,J = 8.3Hz,J′ = 1.4Hz,Ar) ; 7.85 (d,1H,J′ = 1.4Hz,Ar) ; 11.63 (s,1H,exch.$D_2O$,NH).

Example 4

1,3-Dihydro-5-(3,6-dihydro-6-methyl-2-oxo-2H-1,3,4-thiadiazin-5-yl)-3-phenyl-2H-indol-2-one

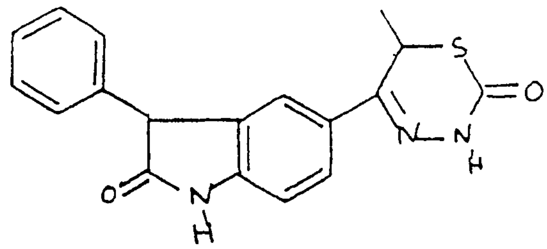

(E4)

0.7g (2,3mmol) 5-(2-chloro-1-oxo-1-propyl)-1,3-dihydro-3-phenyl-2H-indol-2-one (Description 7), 0.4g (3.8mmol) 0-methyl thiocarbazate and 20ml acetonitrile were refluxed for 20 hrs. After evaporation of the solvent, the residue was purified by column chromatography on silica (eluent : hexane/ethyl acetate : 1/1) yielding 0.25g of the desired compound (32%).
m.p. = 165 °C.
IR (KBr)$\nu$ = 3,200 ; 1,715 ; 1,620 ; 1,500 $cm^{-1}$.
NMR ($CDCl_3$) : $\delta$ = 1.63 ppm (d,3H,J = 7.3Hz,$CH_3$-CH) ; 4.27 (q,1H,J = 7.3Hz,CH,-$CH_3$) ; 4.67 (s,1H,øCH) ; 7.00 (d,1H,J = 8.7 Hz,Ar) ; 7.19-7.42 (m,5H,Ar) ; 7.55-7.68 (m,2H,Ar) ; 8.44 (s,1H,exch.$D_2O$,NH) ; 8.89 (s,1H,exch.$D_2O$,NH).

Example 5

1,3-Dihydro-5-(3,6-dihydro-6-methyl-2-oxo-2H-1,3,4-thiadiazin-5-yl)-3,3-dimethyl-1-(phenylmethyl)-2H-indol-2-one

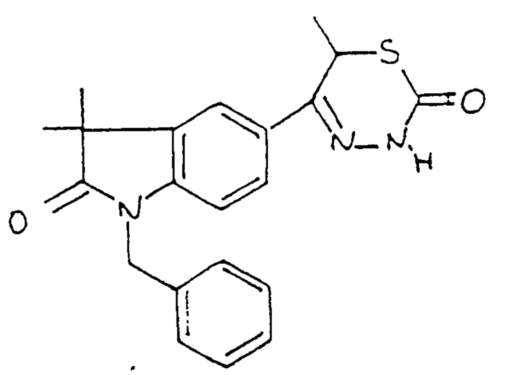

(E5)

2.6g (6.7mmol), 5-(2-bromo-1-oxo-1-propyl)-1,3-dihydro-3,3-dimethyl-1-(phenylmethyl)-2H-indol-2-one (Description 9), 2.5ml acetonitrile, 1.08g 0-methyl thiocarbazate and 1g triethylamine were refluxed for 3 hrs. The solvent was concentrated, the residue taken up in 75ml ethylacetate and washed 3 times with 100ml water. The organic phase was dried over $MgSO_4$ and concentrated to dryness. The residue triturated with ether afforded 0.6g of the desired compound.
m.p. = 190° C.
IR (KBr) $\nu$ = 3,200 ; 1,720 ; 1,635 ; 1,620 ; 1,495 ; 1,385 $cm^{-1}$.
NMR ($CDCl_3$) : $\delta$ = 1.47 ppm (s,6H,$CH_3$-C) ; 1.65 (d,3H,J=7.2Hz,$CH_3$-CH) ; 4.22 (q,1H,J=7.2Hz,CH-$CH_3$) ; 4.94 (s,2H,N-$CH_2$) ; 6.74 (d,1H,J=8.2Hz,Ar) ; 7.23-7.37 (m,5H,Ar) ; 7.43 (dd,1H,J=8.2Hz,J′=1.6Hz,Ar) ; 7.71 (d,1H,J′=1.6Hz,Ar) ; 9.06 (s,1H,exch.$D_2O$,NH).

Example 6

1′-Benzoyl-5′-(3,6-dihydro-6-methyl-2-oxo-2H-1,3,4-thiadiazin-5-yl)-spiro[cyclopropane-1,3′-[3H]indol]-2′-(1′H)-one

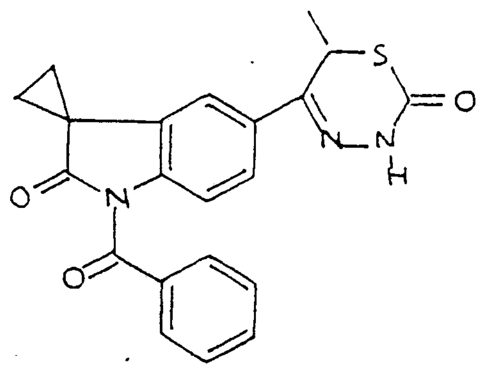

(E6)

(A) 1.69g (5.9mmol) 5′-(3,6-dihydro-6-methyl-2-oxo-2H-1,3,4-thiadiazin-5-yl)-spiro[cyclopropane-1,3′-[3H]indol]-2′-(1′H)-one were dissolved in 20ml DMF and the solution cooled to 0° C. Then, 0.26g (5.9mmol) NaH (55% suspension in oil) were added and the mixture brought to room temperature. 0.83g (5.9mmol) benzoyl chloride were added and the mixture stirred 1hr at room temperature. The mixture was poured in 100ml water and extracted twice with 50ml $CHCl_3$. The organic phase was washed with water, dried over $MgSO_4$ and concentrated to dryness. The residue was chromatographed on a silica column and eluted with hexane/ethyl acetate 1/1.5.
The desired fraction was recrystallised from ethanol giving 0.8g. 51.
m.p. = 252° C.
IR (KBr) $\nu$ = 3,250 ; 1,750 ; 1,680 ; 1,650 ; 1,620 ; 1,290 $cm^{-1}$.
NMR (DMSO-$d_6$) : $\delta$ = 1.50 ppm (d,3H,J=7.1Hz,$CH_3$-CH) ; 1.66-1.86 (2m,2H;2H,cyclopropyl) ; 4.77 (q,1H,J=7.1Hz,CH-$CH_3$) ; 7.46-7.88 (m,8H,Ar) ; 11.70 (s,1H,exch.$D_2O$,NH).

(B) 5-(3,6-Dihydro-6-methyl-2-oxo-2H-1,3,4-thiadiazin-5-yl)-spiro[cyclopropane-1,3′-[3H]indol]-2′-(1′H)-one

0.5g (2mmol) 5-[(2-Chloro-1-oxo)propyl]-spiro[cyclopropane-1,3′-[3]-indol]-2′-(1′H)-one , 0.3g (3.2mmol) methoxy thiocarbonylhydrazine and 6ml acetonitrile were refluxed for 6 hours. The precipitate was isolated by filtration and washed with ether to yield 0.28g.
Yield: 49%
m.p.: 281°C.
IR (KBr) $\nu$ = 3,175; 1,720; 1,620; 1,605; 1,230 $cm^{-1}$.
NMR (DMSO-$d_6$) $\delta$ = 1.54 ppm (m,7H,$CH_3$,$CH_2$-$CH_2$); 4.64 (q,1H,J= 7.2Hz,CH); 6.97 (d,1H,J = 8.2Hz,Ar); 7.40 (d,1H,J′=1.2Hz,Ar); 7.64 (dd,1H,J=8.2,J′=1.2Hz,Ar; 10.70 (s,1H, exch.$D_2O$,NH).

(C) 5-[(2-Chloro-1-oxo)propyl]-spiro[cyclopropane-1,3′-[3H] indol-2′(1′H)-one

4.95g alumminium chloride and 0.8 ml DMF were mixed with stirring at 70°C for 30mins. After cooling 0.6g (3.7mmol) spiro[cyclopropane-1,3′-[3H]-indol]-2′(1′H)-one (m.p.: 190°C; Lit. :180-187°C: JACS 71,2031-5, 1943) and 0.48 g(3.7mmol) 2-chloro propionylchloride were added and stirred at 80°C for 1 hour. The mixture was poured onto 100g ice and 5ml 10 N HCl, then extracted twice with 100ml ethyl acetate.

The organic layer was washed with 2 x 100ml water, dried over magnesium sulphate and evaporated to dryness. Trituration with ether afforded 0.6g (65%) of the title compound.

m.p. = 163°C.

IR (KBr)$\nu$ = 3,000; 1,715; 1,680; 1,615; 1,235; 1.190 $cm^{-1}$.

NMR (DMSO-$d_6$) $\delta$ = 1.74 (m,7H,$CH_3$,$CH_2$-$CH_2$); 5.20 (q,1H,J=6.7Hz,CH); 7.03 (d,1H,J=8.2Hz,Ar); 7.54 (d,1H,J′=1.8Hz,Ar); 7.93 (dd,1H,J=8.2Hz,J′=1.8Hz,Ar); 8.7 (s,1H,exch.$D_2O$,NH).

Example 7

1,3-Dihydro-5-(3,6-dihydro-6-methyl-2-oxo-2H-1,3,4-thiadiazin-5-yl)-3,3-dimethyl-1-phenyl-2H-indol-2-one

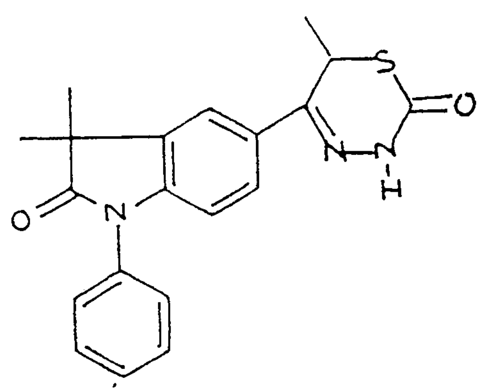

(E7)

1.5g (4mmol) 5-(2-bromo-1-oxo-1-propyl)-1,3-dihydro-3,3-dimethyl-1-phenyl-2H-indol-2-one (Description 12), 0.5g (5mmol) 0-methyl thiocarbazate, 20ml acetonitrile and 1 drop trifluoroacetic acid were heated 6 hrs to reflux. The solvent was then concentrated, the residue dissolved in 50ml ethyl acetate and washed 3 times with water. The organic phase was dried over $MgS0_4$ and concentrated to dryness. The residue triturated with ether afforded 0.45g of the desired compound.

m.p. = 144-5°C.

IR (KBr) $\nu$ = 3,200 ; 1,730 ; 1,620 ; 1,500 ; 1,200 $cm^{-1}$.

NMR ($CDCl_3$) : $\delta$ = 1.53 (s,6H,$CH_3$-C-$CH_3$) ; 1.69 (d,J=7Hz,3H,$CH_3$-CH) ; 4.28 (q,J=7Hz,1H,CH-$CH_3$) ; 6.89 (d,1H,Ar) ; 7.5 (m,6H,Ar) ; 7.76 (d,1H,Ar) ; 8.79 (s,1H,exch.$D_20$,NH).

Example 8

1,3-Dihydro-5-(3,6-dihydro-6-methyl-2-oxo-2H-1,3,4-thiadiazin-5-yl)-3,3-dimethyl-1-(2-phenylethyl)-2H-indol-2-one

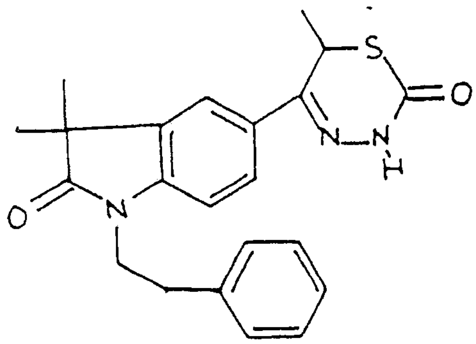

(E8)

4g (10mmol) 5-(2-bromo-1-oxo-1-propyl)-1,3-dihydro-3,3-dimethyl-1-(2-phenylethyl)-2H-indol-2-one (Description 14), 1.07g (10mmol) 0-methyl thiocarbazate, 20ml acetonitrile and 1g triethylamine were refluxed 4 hours. The solvent was then concentrated, the residue dissolved in 100ml ethyl acetate and washed 3 times with water. The organic phase was dried over $MgSO_4$, and concentrated to dryness. The product was purified by two consecutive column chromatography on silica using methylene chloride/ethyl acetate : 5/1 as eluent, then with hexane/ethyl acetate : 1/1.
The solid was recrystallized in ether, yielding 250mg of the desired compound.
m.p. 166°C.
IR (KBr) $\nu$ = 3,200 ; 3,100 ; 2,970 ; 2,930 ; 1,693 ; 1,647 1,617 ; 1,360 ; 1,160 $cm^{-1}$.
NMR ($CDCl_3$) : $\delta$ = 1.30 (s,6H,2 x $CH_3$) ; 1.65 (d,3H,J = 7Hz,$CH_3$-CH) ; 3.02 and 3.97 (2t,J = 8Hz,2 x 2H,Ar $CH_2CH_2N$) ; 4.24 (q,J = 7Hz,1H,$CH_3$-CH) ; 6.8-7.7 (m,8H,Ar) ; 8.89 (s,1H,exch.$D_2O$,NH).

Example 9

1-Cyclohexylmethyl-5-(3,6-dihydro-6-methyl-2-oxo-2H-1,3,4-thiadiazin-5-yl)-1,3-dihydro-3,3-dimethyl-2H-indol-2-one

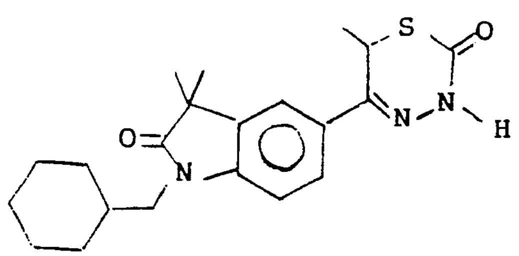

E9

4g (10.2mmol) 5-(2-bromo-1-oxopropyl,-1-cyclohexylmethyl-1,3-dihydro-3,3-dimethyl-2H-indol-2-one (Description 16), 40ml acetonitrile, 2g (13.5mmol) O-methyl thiocarbonate and 1.02g (10.2mmol) triethylamine were refluxed under argon for 2 hours. The reaction mixture was concentrated to dryness, dissolved in 100ml ethyl ether, washed with water (2 x 100ml), 1N hydrochloric acid (100ml) and water again (2 x 100ml) and then dried over magnesium sulphate. After concentration, the residue was triturated with ethyl ether to give 1.80g (46%) of the desired compound.
m.p. 194°C
IR (KBr): $\nu$ = 3200; 2930; 1715; 1635; 1620; 1490; 1385$cm^{-1}$.
NMR ($CDCl_3$): $\delta$ = 0.9-1.9 (m, 20H, cyclohexane (11H) and including s at 1.41 ppm, 6H, $(CH_3)_2C$, and d at 1.69 ppm, J = 7Hz, 3H, $CH_3CH$); 3.56 (d,J = 7Hz,2H,N-$CH_2$); 4.28 (q,J = 7Hz,1H,$CH_3CH$); 6.89 (d,J = 8Hz,1H,Ar); 7.56 (dd,J = 8Hz,J′ = 1.8Hz,1H,Ar); 7.69(d,J′ = 1.8Hz,Ar); 9.00 (s,1H,exch.$D_2O$,NH).

Description 17

Cyclohexanacetic acid, phenylhydrazide

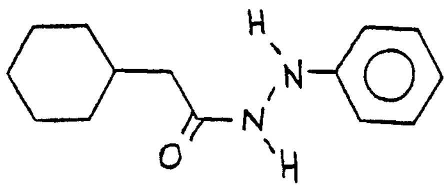

(D17)

10.8g phenylhydrazine were dissolved in 100ml DMF containing 7.8g pyridine, and 16g cyclohexyl acetyl chloride were added dropwise at 5°C. The stirring was continued for one hour, the solution reduced by half, and poured into 150ml water. The solid obtained after filtration was washed with 30ml ethanol and 50ml ether.
Yield : 64%
m.p. : 202°C
IR (KBr) : $\nu$ = 3,310 ; 3,230 ; 3,050 ; 2,940 ; 1,640 ; 1,605 $cm^{-1}$.
NMR (DMSO-$d_6$) : $\delta$ = 0.8-1.7 (m,11H,cyclohexyl); 2.03 (d,2H,J=6.8Hz,$CH_2CO$); 6.6-7.2 (m,5H,Ar); 7.68 (d,1H,J=2.8Hz,exch.$D_2O$,NH); 9.58 (d,1H,J=2.8Hz,exch.$D_2O$,NH).

Description 18

3-Cyclohexyl-1,3-dihydro-2H-indol-2-one

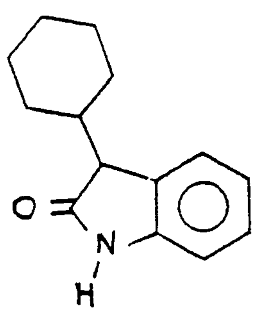

(D18)

5g cyclohexanacetic acid, phenylhydrazide (D17) and 2g calcium hydride were heated to 220°C for 2 hours. After cooling, 10ml 2N hydrochloric acid were carefully added. The solution was extracted with ethyl acetate (2 x 50ml). The combined organic phases were washed with water (2 x 30ml) and dried over magnesium sulphate. The residue was triturated with diethyl ether.
Yield : 26%
m.p. : 170°C
IR (KBr) : $\nu$ = 3,200 ; 2,940 ; 1,702 ; 1,618 $cm^{-1}$.
NMR (DMSO-$d_6$) : $\delta$ = 1.0-2.1 (m,11H,cyclohexane); 3.30 (d,1H,J=3.4Hz,CH-cyclohexane); 6.7-7.3 (m,4H,Ar); 10.32 (s,1H,exch.$D_2O$,NH).

Description 19

5-(2-Bromo-1-oxopropyl)-3-cyclohexyl-1,3-dihydro-2H-indol-2-one

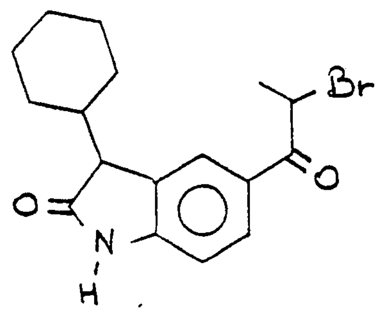

(D19)

1.07g 3-cyclohexyl-1,3-dihydro-2H-indol-2-one (D18) were added to a mixture of 1ml DMF and 7g aluminum chloride. 1.08g 2-bromo propionyl bromide were added dropwise while maintaining the temperature below 20°C. The mixture was then heated to 60°C during one hour and poured on 50g ice containing 5ml conc. hydrochloric acid. The solution was extracted with ethyl acetate (2 x 40ml). The combined organic phases were washed with water, dried over magnesium sulfate and concentrated to dryness. The residue was triturated with diethyl ether.
Yield : 74%
m.p. : 230°C
IR (KBr) : $\nu$ = 3,200 ; 1,721 ; 1,672 ; 1,615 $cm^{-1}$.
NMR ($DMSO-d_6$) : $\delta$ = 1.0-2.1 (m,14H,cyclohexane,11H,+d at 1.76ppm,J=6.4Hz,3H,$CH_3CH$); 3.50 (m,1H,CH cyclohexane);5.82 (m,1H,$CHCH_3$); 6.93 (d,J=8.1Hz,2H,Ar); 7.90 (s,1H,Ar); 7.96 (d,J=8.1Hz,2H,Ar); 10.84 (s,1H,exch.$D_2O$,NH).

Description 20

5-(2-Chloro-1-oxoethyl)-1,3-dihydro-3-phenylthio-2H-indol-2-one

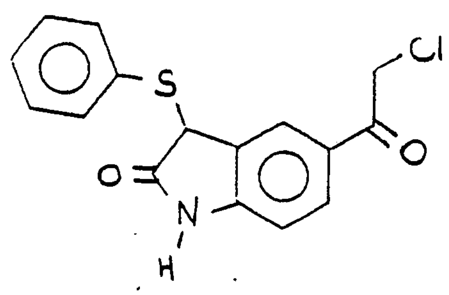

(D20)

3.84g t-butyl hypochlorite were added dropwise to a well stirred solution of 6g 1-(4-aminophenyl)-2-chloro-ethanone in 100ml methylene chloride at -65°C. After 20 minutes at -65°C, 6.9g ethyl phenylthioacetate were added to the solution. Stirring was continued at the same temperature for $2\frac{1}{2}$ hours and 3.6g triethylamine were then added. The solution was stirred 30 minutes at room temperature. The solution was washed with water, concentrated and the residue dissolved in 100ml ether containing 18ml 2N hydrochloric acid. The mixture was stirred overnight, then washed with water (2 x 30ml), dried over magnesium sulphate and concentrated. The compound was purified by column chromatography on silica (eluent : hexane/ethyl acetate : 2/1).
Yield : 12%
m.p. : 75°C
IR (KBr) : $\nu$ = 3,200 ; 1,725 ; 1,680 ; 1,615 $cm^{-1}$.
NMR ($DMSO-d_6$) : $\delta$ = 5.13 (m,3H,$CH_2Cl$ + CHSAr); 6.8-8.0(m,8H,Ar); 10.94 (s,1H,exch.$D_2O$,NH).

Description 21

5-(2-Chloro-1-oxoethyl)-3-cyclohexyl-1,3-dihydro-2H-indol-2-one

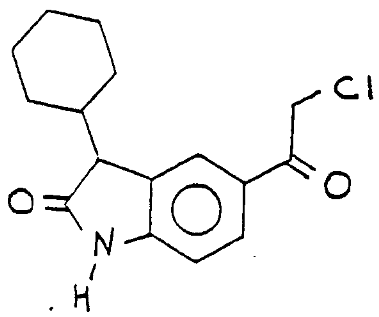

(D21)

Starting from 3-cyclohexyl-1,3-dihydro-2H-indol-2-one (D18) and chloroacetyl chloride, and following the method described in D19, the desired compound was obtained.
Yield : 49%
m.p. : 165°C
IR (KBr) : $\nu$ = 3,250 ; 2,930 ; 1,710 ; 1,615 $cm^{-1}$.
NMR (DMSO-$d_6$) : $\delta$ = 1.0-2.1 (m,11H,cyclohexyl); 3.44 (d,1H,J=3.1Hz,CH-cyclohexyl); 5.13 ($A_2B_2$,J=21.0Hz,J=15.6Hz,$CH_2CO$); 6.92 (d,1H,J=8.1Hz,Ar); 7.83 (s,1H,Ar); 7.89 (d,1H,J=8.1Hz,Ar); 10.82 (s,1H,exch.$D_2O$,NH).

Description 22

5-(2-Chloro-1-oxo propyl)-1,3-dihydro-3-methyl-3-phenylthio-2H-indol-2-one

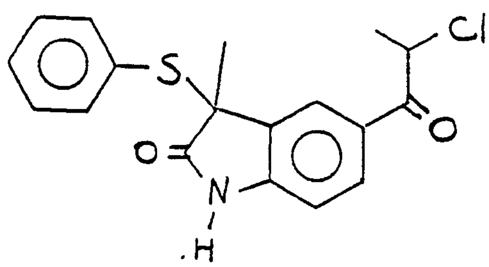

(D22)

Starting from 1-(4-aminophenyl)-2-chloropropanone and following the method given in D20, the desired compound was obtained.
Yield : 28%
m.p. : 195°C
IR (KBr) : $\nu$ = 3,100 ; 1,725 ; 1,675 ; 1,610 $cm^{-1}$.
NMR (DMSO-$d_6$) : $\delta$ = 1.62 (m,6H,$CH_3CH+CH_3C$); 5.78 (m,1H,$CH_3$ CH); 6.75 (m,1H,Ar indole); 7.1-7.3 (m,5H,ArS); 7.8-8.1 (m,2H,Ar indole); 10.80 (m,1H,exch.$D_20$,NH).

Description 23

1,3-Dihydro-3,3-di-(4-methylphenyl)-5-propyl-2H-indol-2-one

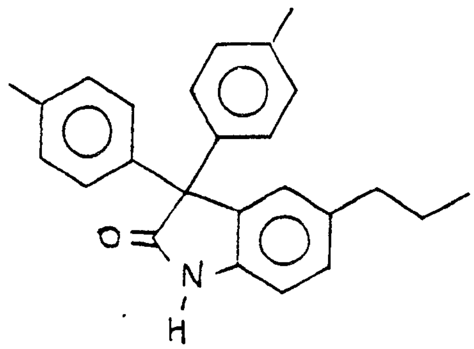

(D23)

5ml concentrated sulphuric acid were added to a solution of 7g 5-propyl-indole-2,3-dione in 100ml toluene and the solution was heated for 4 hours at 80°C. The desired compound crystallized on cooling.
Yield : 53%
m.p. : 203-6°C
IR (KBr) : $\nu$ = 3,170 ; 1,700 ; 1,620 $cm^{-1}$.
NMR (DMSO-$d_6$) : $\delta$ = 0.85 (t,3H,J=7.4Hz,$CH_3CH_2$); 1.5 (m,2H,$CH_3CH_2$); 2.26 (s,6H,2 x $CH_3$Ar); 2.47 (t,2H,J=8.0Hz,$CH_2$Ar); 6.8-7.15 (m,11H,Ar); 10.59 (s,1H,exch.$D_2O$,NH).

Description 24

1,3-Dihydro-3,3-di-(4-methylphenyl)-5-(1-oxopropyl)-2H-indol-2-one

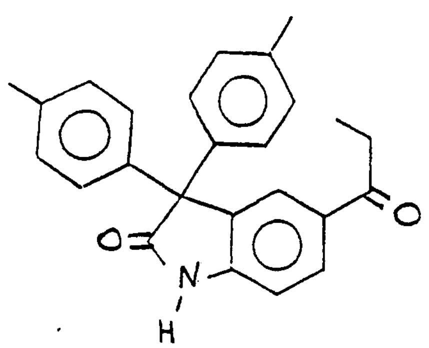

(D24)

A solution of 3.96g chromium (VI) oxide, 32ml water and 12.2ml acetic acid was added at <8°C to a solution of 7g 1,3-dihydro-3,3-di-(4-methylphenyl)-5-propyl-2H-indol-2-one (D23) in 8.9ml acetic acid and 2.8ml acetic anhydride.The solution was then stirred at 40°C overnight. Filtration over silica, after concentration by a column chromatography, (silica, eluent : hexane/ethyl acetate : 3/2) afforded the desired product.
Yield : 29%
m.p. : 265°C
IR (KBr) : $\nu$ = 3,175 ; 1,710 ; 1,682; 1,615 $cm^{-1}$.
NMR (DMSO-$d_6$) : $\delta$ = 1.03 (t,3H,J=7.1Hz,$CH_3CH_2$); 2.27 (s,6H,2 x $CH_3$ tolyl); 2.95 (q,2H,J=7.1Hz,$CH_3CH_2$); 7.00-7.20 (m,9H,8H toluene + 1H indole); 7.72 (s,1H,Ar indole); 7.95 (d,1H,J=8.2Hz,Ar indole); 11.11 (s,1H,exch.$D_2O$,NH).

Description 25

5-(2-Bromo-1-oxopropyl)-1,3-dihydro-3,3-di-(4-methylphenyl)-2H-indol-2-one

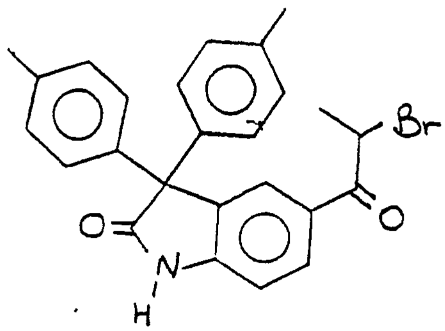

(D25)

0.86g bromine dissolved in 10ml chloroform were added dropwise to a solution of 1,3-dihydro-3,3-di-(4-methylphenyl)-5-(1-oxopropyl)-2H-indol-2-one (D24) in 40ml chloroform and 10ml dioxan. At the end of the addition, 50ml chloroform were added, the solution was washed with 3 x 100ml water, and dried over magnesium sulphate. After concentration, the residue was triturated in diethyl ether.
Yield : 86%
m.p. : 150°C
IR (KBr) : $\nu$ = 3,250 ; 1,732 ; 1,682; 1,615 $cm^{-1}$.
NMR (DMSO-$d_6$) : $\delta$ = 1.73 (d,3H,J=6.4Hz,$CH_3CH$); 2.27 (s,6H,2 x $CH_3$ Ar); 5.75 (q,1H,J=6.4Hz,$CH_3CH$); 7.00-7.20 (m,9H,8H toluen + 1H indole); 7.83 (s,1H,Ar indole); 8.03 (d,1H,J=8.2Hz,Ar indole); 11.21 (s,1H,exch.$D_2O$,NH).

Description 26

S-Ethyl 2,3-dihydro-3,3-dimethyl-5-(1-oxopropyl)-2-oxo-1H-indole-1-carbothioate

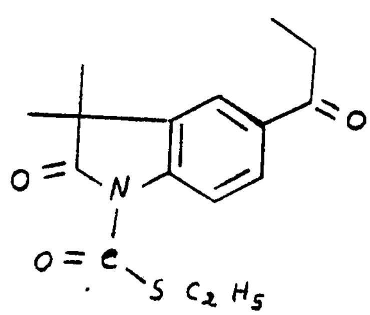

(D26)

4.34g (20mmol) 1,3-dihydro-3,3-dimethyl-5-(1-oxopropyl)-2H-indol-2-one (D1 ) in 100ml DMF were cooled at 0°C. 0.92g (21mmol) sodium hydride from a 55% dispersion in mineral oil was added and the mixture was stirred at 0°C for 0.5 hours then 0.5 hours at 20°C. After cooling to 0°C 2.62g (21mmol) S-ethyl chloro thioformate was added dropwise. The reactional mixture was stirred at room temperature for 2hr and the solvent evaporated under vacuum. The residue was taken up in methylene chloride, washed with water then with brine and dried over $MgSO_4$. The solvent was evaporated and the compound purified by trituration in diisopropyl ether.
Yield : 4.62g (76%)
m.p. : 146°C
IR (KBr) : $\nu$ = 1,765 ; 1,660 ; 1,610; 1,470; 1,200 $cm^{-1}$.
NMR ($CDCl_3$) : $\delta$ = 1.24 (t,J=7.3Hz,3H,$CH_3$-$CH_2$); 1.39 (t,J=7.4Hz,3H,$CH_3$-$CH_2$); 1.48 (s,6H,$(CH_3)_2$-C); 3.04 (q,J=7.3Hz,2H,$CH_3$-$CH_2$); 3.05 (q,J=7.4Hz,2H,$CH_3$-$CH_2$); 7.89 (d,J=1.6Hz,1H,Ar); 7.92 (dd,J=1.6Hz,J′=8.6Hz,1H,Ar); 8.28 (d,J=8.6Hz,1H,Ar).

Description 27

S-Ethyl 5-[(2-bromo-1-oxo)propyl]-2,3-dihydro-3,3-dimethyl-2-oxo-1H-indole-1-carbothioate

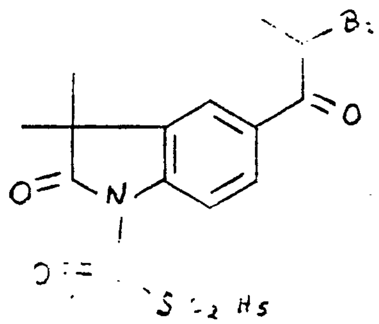

(D27)

A solution of 4.0g (13.1mmol) S-ethyl-2,3-dihydro-3,3-dimethyl- 5-(1-oxopropyl)-2-oxo-1H-indole-1-carbothioate (D26) in 120ml chloroform and 6ml 1,4-dioxane was heated at 35-40°C. 2.09g (13.1mmol) bromine in 5ml chloroform were added dropwise and stirring was maintained until bromine coloration disappeared. 200ml methylene chloride was added and the resulting organic solution was washed with water (2 x100ml) then with brine (100ml) and dried over Mg $SO_4$. The solvent was evaporated to dryness leaving 5g (99%) of crude compound used directly in the next step.

Description 28

2,2,2-Trifluoroethyl 2,3-dihydro-3,3-dimethyl-5-(1-oxo-propyl)-2-oxo-1H-indole-1-carboxylate

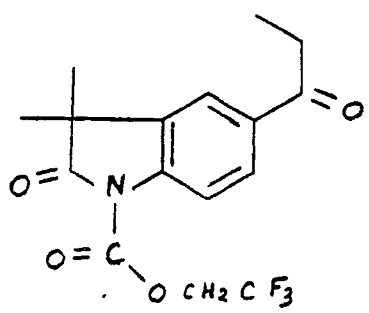

(D28)

Starting from 1,3-dihydro-3,3-dimethyl-5-(1-oxopropyl)-2H-indol-2-one (D1) and 2,2,2-trifluoroethyl chloroformate (see PPG Industries, Inc. US Patent No. 3,852,464), and following the procedure of Description 26 with a reaction time of 4 hours at room temperature instead of 2 hours, afforded the title compound after trituration in diisopropyl ether.

Yield : 60.4%

m.p. : 130°C

IR (KBr) : $\nu$ = 1,810 ; 1,685 ; 1,615; 1,325; 1,180 $cm^{-1}$.

NMR ($CDCl_3$) : $\delta$ = 1.25 (t,J=7.3Hz,3H,$CH_3$-$CH_2$); 1.50 (s,6H,$(CH_3)_2C$); 3.01 (q,J=7.3Hz,2H,$CH_3$-$CH_2$); 4.79 (q,J=8.1Hz,2H,$CF_3$-$CH_2$); 7.90 to 7.95 (m,3H,Ar).

Description 29

2,2,2-Trifluoroethyl 5-[(2-bromo-1-oxo)propyl]-2,3-dihydro-3,3-dimethyl-2-oxo-1H-indole-1-carboxylate

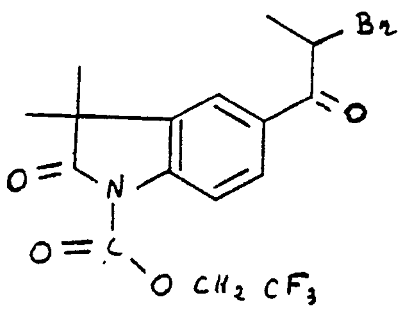

(D29)

Starting from 2,2,2-trifluoroethyl 2,3-dihydro-3,3-dimethyl-5-(1-oxopropyl)-2-oxo-1H-indole-1-carboxylate (D28) and following the procedure of Description 27 with a reaction carried out at 50°C instead of 35-40°C, the title compound was obtained after trituration for 15hr in cyclohexane. It contained some starting material but was used without further purification in the next step.
Yield : 81%
NMR ($CDCl_3$) : $\delta$ = 1.51 (s,6H,$(CH_3)_2C$); 1.92 (d,J=6.6Hz,3H,$CH_3$-CH); 4.79 (q,J=8.1Hz,2H,$CF_3$-$CH_2$); 5.29 (q,J=6.6Hz,1H,$CH_3$-CHBr); 7.90 to 8.07 (m,3H,Ar).

Description 30

2-Methoxyethyl 2,3-dihydro-3,3-dimethyl-5-(1-oxopropyl)-2-oxo-1H-indole-1-carboxylate

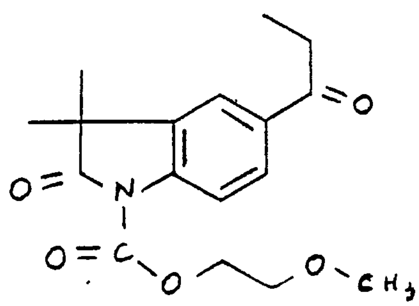

(D30)

3.04g (14mmol) 1,3-dihydro-3,3-dimethyl-5-(1-oxopropyl)-2H-indol-2-one (D1) in 40ml DMF were cooled at 0°C. 0.67g (15.4mmol) sodium hydride from a 55% dispersion in mineral oil was added and the mixture was stirred at room temperature for 2 hours. After cooling again at 0°C 2.9g (16.8mmol) 2-methoxyethyl chloroformate (J.R. Geigy, US Patent No. 2,573,420) was added dropwise and stirring was maintained at 5 to 10°C for 0.5 hour. The solvent was evaporated under vacuo and the residue taken up in water. The crystallized crude compound was filtered off, washed with water, dried and used in the next step without further purification.
Yield : 3.7g (82.7%)
m.p. : 144°C
IR (KBr) : $\nu$ = 1,780; 1,725; 1,680; 1,615; 1,305; 1,200 $cm^{-1}$.
NMR ($CDCl_3$) : $\delta$ = 1.24 (t,J=7.3Hz,3H,$CH_3$-$CH_2$); 1.47 (s,6H,$(CH_3)_2C$); 3.01 (q,J=7.3Hz,2H,$CH_3$-$CH_2$); 3.46 (s,3H,$CH_3O$); 3.78(t,J=4.6Hz,2H,$CH_2$-$CH_2$); 4.57 (t,J=4.6Hz,2H,$CH_2$-$CH_2$); 7.8 to 7.97 (m,3H,Ar).

Description 31

2-Methoxyethyl 5-[(2-bromo-1-oxo)propyl]-2,3-dihydro-3,3-dimethyl-2-oxo-1H-indole-1-carboxylate

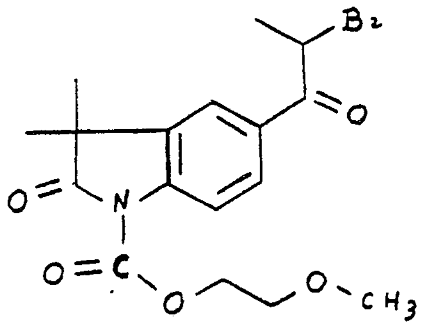

(D31)

Starting from 2-methoxyethyl 2,3-dihydro-3,3-dimethyl-5-(1-oxopropyl)-2-oxo-1H-indole-1-carboxylate (D30) and following the procedure of Description 27, the title compound was obtained.
Yield : 95.8%
m.p. : 123°C
NMR ($CDCl_3$) : $\delta$ = 1.48 (s,6H,$(CH_3)_2C$); 1.92 (d,J=6.6Hz,3H,$CH_3$-CH); 3.46 (s,3H,$CH_3O$); 3.78-(t,J=4.6Hz,2H,$CH_2$-$CH_2$); 4.57 (t,J=4.6Hz,2H,$CH_2$-$CH_2$); 5.29 (q,J=6.6Hz,1H,$CH_3$-CHBr); 7.93 to 8.01 (m,3H,Ar).

Description 32

2-(Methylthio)ethyl chloroformate

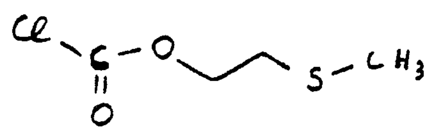

(D32)

6.08g (66mmol) (2-methylthio)ethanol in solution in 20ml dry diethyl ether were added dropwise to a solution of 14.8g (50mmol) bis (trichloromethyl) carbonate in 60ml dry diethyl ether, and stirred at 0°C. After 5 minutes stirring, 5.8g (77mmol) pyridine were added taking care that the temperature did not rise above 5°C. The temperature was then allowed to reach room temperature and stirring maintained for 16 hours. The hydrochloric acid was eliminated by passing a current of argon through the reaction mixture before pouring it onto water. The aqueous phase was extracted with diethyl ether (100ml). The organic solutions were mixed, washed with water, with a saturated aqueous $NaHCO_3$ solution, with water and dried over $MgSO_4$.The solvent was evaporated to afford the desired compound which was used in the next step without further purification.
Yield : 7.36g (72%)
IR (film) $\nu$ = 1,775; 1,140 $cm^{-1}$.

Description 33

2-(Methylthio)ethyl 2,3-dihydro-3,3-dimethyl-5-(1-oxopropyl)-2-oxo-1H-indole-1-carboxylate

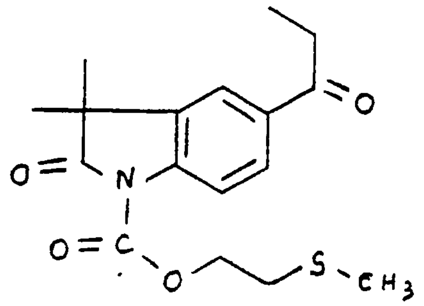

(D33)

Starting from 1,3-dihydro-3,3-dimethyl-5-(1-oxopropyl)-2H-indol-2-one (D1 ) and 2-(methylthio)ethyl chloroformate (D32) and following the procedure of Description 30 with a reaction time of 3.5 hours at room temperature then 2 days at 5°C instead of 0.5 hours at 5 to 10°C, the desired compound was obtained after purification by chromatography on silica (methylene chloride/ethyl acetate : 9/1) and trituration in diisopropyl ether.

Yield : 38.4%

m.p. : 148°C

IR (KBr) : $\nu$ = 1,785; 1,720; 1,680; 1,615; 1,310; 1,200 1,025 $cm^{-1}$.

NMR ($CDCl_3$) : $\delta$ = 1.24 (t,J=7.2Hz,3H,$CH_3$-$CH_2$); 1.47 (s,6H,$(CH_3)_2C$); 2.23 (s,3H,$CH_3S$); 2.92 (t,J=6.9Hz,2H,$CH_2$-$CH_2$); 3.01 (q,J=7.2Hz,2H,$CH_3$-$CH_2$); 4.58 (t,J=6.9Hz,2H,$CH_2$-$CH_2$); 7.87 to 7.99 (m,3H,Ar).

Description 34

2-(Methylthio)ethyl 5-[(2-bromo-1-oxo)propyl]-2,3-dihydro-3,3-dimethyl-2-oxo-1H-indole-1-carboxylate

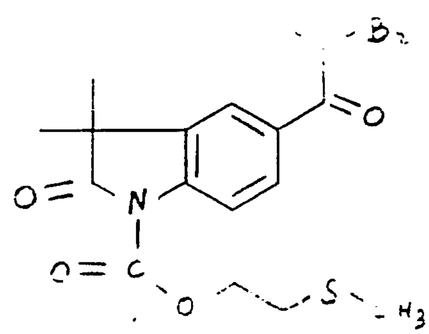

(D34)

Starting from 2-(methylthio)ethyl 2,3-dihydro-3,3-dimethyl-5-(1-oxopropyl)-2-oxo-1H-indole-1-carboxylate (D33) and following the procedure of Description 27, the title compound was obtained in a form sufficiently pure for the next step.

Yield : 96%

m.p. : 102°C

NMR ($CDCl_3$) : $\delta$ = 1.48 (s,6H,$(CH_3)_2C$); 1.92 (d,J=6.6Hz,3H,$CH_3$-CHBr); 2.23 (s,3H,$CH_3S$); 2.92 (t,J=6.9Hz,2H,$CH_2$-$CH_2$); 4.59 (t,J=6.9Hz,2H,$CH_2$-$CH_2$); 5.29 (q,J=6.6Hz,1H,$CH_3$CHBr); 7.93 to 8.03 (m,3H,Ar).

Description 35

Ethyl 2,3-dihydro-3,3-dimethyl-5-(1-oxopropyl)-2-oxo-1H-indole-1-sulfinate

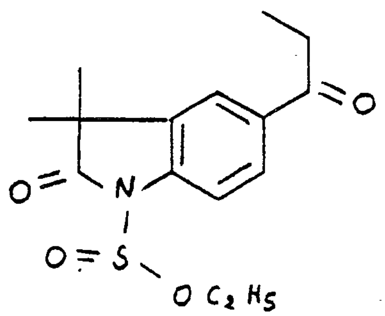

(D35)

3.26g (15mmol) 1,3-dihydro-3,3-dimethyl-5-(1-oxopropyl)-2H-indol-2-one (D1 ) in 50ml DMF were cooled at 0°C. 0.70g (16mmol) sodium hydride from a 55% dispersion in mineral oil was added and the mixture was stirred for 0.5 hours at 0°C then at room temperature until hydrogen evolution ceased. After cooling at 0°C, 2.1g (16mmol) ethyl chlorosulfinate were added dropwise. The reaction mixture was stirred at room temperature for 1.5 hours. The solvent was evaporated under vacuo and the resulting oily residue was taken up in water, where it crystallized. The solid was filtered off, washed with water, dried and used in the next step without further purification.
Yield : 4.48g (96%)
m.p. : 123°C
IR (KBr) : $\nu$ = 1,770; 1,680; 1,615; 1,370; 1,200; 1,165 $cm^{-1}$.
NMR ($CDCl_3$) : $\delta$ = 1.24 (t,J=7.2Hz,3H,$CH_3$-$CH_2$); 1.41 (t,J=7.5Hz,3H,$CH_3$-$CH_2$); 1.49 (s,6H,$(CH_3)_2C$); 3.00 (q,J=7.2Hz,2H,$CH_3$-$CH_2$); 3.62 (q,J=7.5Hz,2H,$CH_3CH_2O$); 7.88 to 7.91 (m,3H,Ar).

Description 36

Ethyl 5-[(2-bromo-1-oxo)propyl]-2,3-dihydro-3,3-dimethyl-2-oxo-1H-indole-1-sulfinate

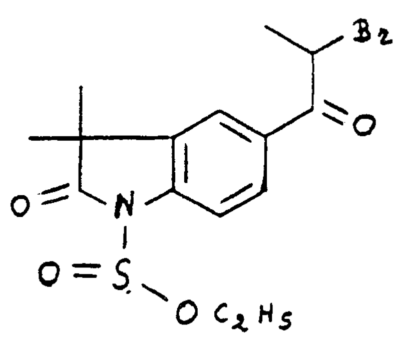

(D36)

Starting from ethyl 2,3-dihydro-3,3-dimethyl-5-(1-oxopropyl)-2-oxo-1H-indole-1-sulfinate (D35) and following the procedure of Description 27, the title compound was obtained after trituration in diisopropyl ether.
Yield : 74%
m.p. : 128.5°C
IR (KBr) : $\nu$ = 1,760; 1,680; 1,610; 1,360; 1,165 $cm^{-1}$.
NMR ($CDCl_3$) : $\delta$ = 1.41 (t,J=7.4Hz,3H,$CH_3CH_2$); 1.50 (s,6H,$(CH_3)_2C$); 1.92 (d,J=6.6Hz,3H,$CH_3CHBr$); 3.63 (q,J=7.4Hz,2H,$CH_3CH_2O$); 5.27 (q,J=6.6Hz,1H,$CH_3$-$CHBr$); 7.87 to 8.02 (m,3H,Ar).

Description 37

Cyclohexyl 2,3-dihydro-3,3-dimethyl-5-(1-oxopropyl)-2-oxo-1H-indole-1-carboxylate

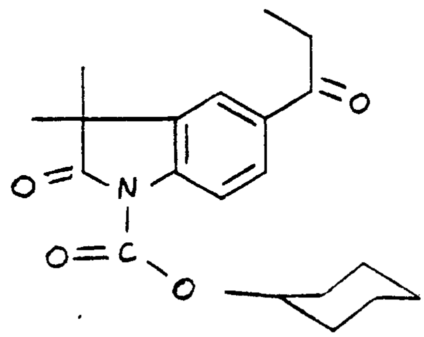

(D37)

Starting from 1,3-dihydro-3,3-dimethyl-5-(1-oxopropyl)-2H-indol-2-one (D1 ) and cyclopropyl chloroformate (Y.Iwakura and A.Nabeya, J.Org.Chem.,1960,25,1118) and following the procedure of Description 35 with a reaction time of only 1 hour instead of 1.5 hours the title compound was obtained.
Yield : 91%
m.p. : 144-5°C
IR (KBr) : $\nu$ = 1,775; 1,725; 1,680; 1,615; 1,200 $cm^{-1}$.
NMR ($CDCl_3$) : $\delta$ = 1.24 (t,J=7.2Hz,3H,$CH_3CH_2$); 1.27 to 1.99 (m,16H,-$(CH_2)_5$- and $(CH_3)_2C$); 3.01 (q,J=7.2Hz,2H,$CH_3$-$CH_2$); 5.06 (m,1H,OCH); 7.87 to 7.95 (m,3H,Ar).

Description 38

Cyclohexyl 5-[(2-bromo-1-oxo)propyl]-2,3-dihydro-3,3-dimethyl-2-oxo-1H-indole-1-carboxylate

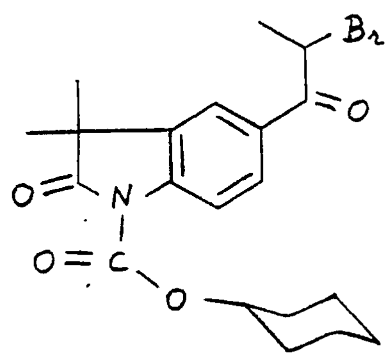

(D38)

Starting from cyclohexyl 2,3-dihydro-3,3-dimethyl-5-(1-oxopropyl)-2-oxo-1H-indole-1-carboxylate (D37) and following the procedure of Description 27, the title compound was obtained after trituration in ligroin.
Yield : 63%
m.p. : 140-1°C
IR (KBr) : $\nu$ = 1,780; 1,720; 1,680; 1,610; 1,220 $cm^{-1}$.
NMR ($CDCl_3$) : $\delta$ = 1.40 to 2.03 (m,19H,(-$CH_2$)$_5$-(10H) and including s at 1.47 ppm, 6H,$(CH_3)_2C$ and d at 1.92 ppm, J=6.6Hz,3H,$CH_3CH$); 5.07 (m,1H,O-CH); 5.29 (q,J=6.6Hz,1H,$CH_3CHBr$); 7.92 to 8.01 (m,3H,Ar).

Description 39

2-Cyanoethyl 2,3-dihydro-3,3-dimethyl-5-(1-oxopropyl)-2-oxo-1H-indole-1-carboxylate

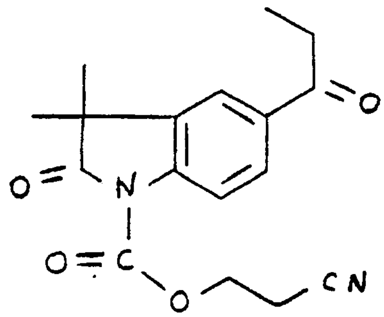

(D39)

Starting from 1,3-dihydro-3,3-dimethyl-5-(1-oxopropyl)-2H-indol-2-one (D1) and 2-cyanoethyl chloroformate (V.I. Kondratenko and I.G.Khaskin, Zh.Org.Khim.,1970,6,2223) and following the procedure of Description 35 with a reaction time of 16 hours instead of 1.5 hours, the title compound was obtained after purification by chromatography on silica (chloroform/methanol : 98/2) then trituration in ligroin.
Yield : 33%
m.p. : 154-5°C
IR (KBr) : $\nu$ = 2,250; 1,710; 1,680; 1,610; 1,495; 1,345 1,200 $cm^{-1}$.
NMR ($CDCl_3$) : $\delta$ = 1.24 (t,J=7.3Hz,3H,$CH_3CH_2$); 1.43 (s,6H,$(CH_3)_2C$); 2.79 (t,J=6.8Hz,2H,$CH_2CN$); 2.99 (q,J=7.3Hz,2H,$CH_3$-$CH_2$); 4.06 (t,J=6.8Hz,2H,O $CH_2CH_2$); 6.98 (d,J=8.2Hz,1H,Ar); 7.89 (d,J′=1.3Hz,1H,Ar); 7.96 (dd,J=8.2Hz,J′=1.3Hz,1H,Ar).

Description 40

2-Cyanoethyl 5-[(2-bromo-1-oxo)propyl]-2,3-dihydro-3,3-dimethyl-2-oxo-1H-indole-1-carboxylate

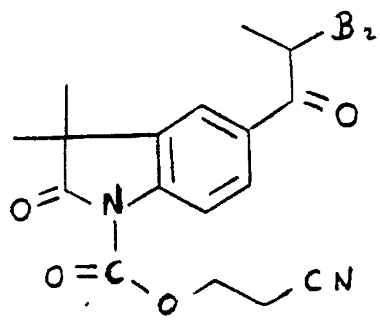

(D40)

Starting from 2-cyanoethyl 2,3-dihydro-3,3-dimethyl-5-(1-oxopropyl)-2-oxo-1H-indole-1-carboxylate (D39) and following the procedure of Description 27, the title compound was obtained after trituration in ligroin.
Yield : 83%
m.p. : 130-1°C
IR (KBr) : $\nu$ = 2,250; 1,710; 1,680; 1,610; 1,495; 1,345 1,200 $cm^{-1}$.
NMR ($CDCl_3$) : $\delta$ = 1.44 (s,6H,$(CH_3)_2C$); 1.91 (d,J=6.6Hz,3H,$CH_3$-CH); 2.80 (t,J=6.8Hz,2H,$CH_2CN$); 4.07 (t,J=6.8Hz,2H,$OCH_2$); 5.28 (q,J=6.6Hz,1H,$CH_3$-CHBr); 7.01 (d,J=8.3Hz,1H,Ar); 7.94 (d,J′=1.6Hz,1H,Ar); 8.03 (dd,J=8.3Hz,J′=1.6Hz,1H,Ar).

Description 41

Ethyl 3-[(4-chlorophenyl)methyl]-2,3-dihydro-2-oxo-1H-indole-3-carboxylate

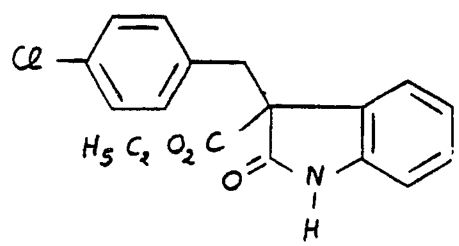

(D41)

1.3g (56mmol) sodium ethylate in 30ml ethanol were added to a solution of 9.75g (50mmol) ethyl 2,3-dihydro-2-oxo-1H-indole-3-carboxylate (M.J. Kornet, A.P. Thio and J.H. Thorstenson, J.Pharm.Sci.,1977,66,1022) in 170ml ethanol. After stirring for 0.5 hours at room temperature, a solution of 8.85g (55mmol) 4-chlorobenzyl chloride in 10ml ethanol was added dropwise. The mixture was stirred 19 hours at room temperature then 3 hours at 50-60°C. The solvent was evaporated and the residue taken up in 500ml diethyl ether. The organic solution was washed with water (2 x 200ml), dried over $MgSO_4$ and concentrated to dryness. The crude compound obtained was purified by chromatography on silica (methylene chloride/ethyl acetate : 9/1) to afford the desired compound.
Yield : 8.4g (50%)
m.p. : 153°C
IR (KBr) : $\nu$ = 1,740; 1,720; 1,680; 1,620; 1,475; 1,230 $cm^{-1}$.
NMR ($CDCl_3$) : $\delta$ = 1.07 (t,J=7.2Hz,3H,$CH_3$-$CH_2$); 3.35 and 3.51 (2d,J=13.2Hz,2H,$CH_2$Ar); 4.10 (q,J=7.2Hz,2H,$CH_3CH_2O$); 6.66 (d,J=7.5Hz,1H,Ar); 6.87 (d,J=8.4Hz,2H,Ar); 6.98 (t,J=7.5Hz,1H,Ar); 7.12 (d,J=8.4Hz,2H,Ar); 7.16 (t,J=7.5Hz,1H,Ar); 7.37 (d,J=7.5Hz,1H,Ar); 10.48 (s,1H,NH).

Description 42

3-[(4-chlorophenyl)methyl]-1,3-dihydro-2H-indol-2-one

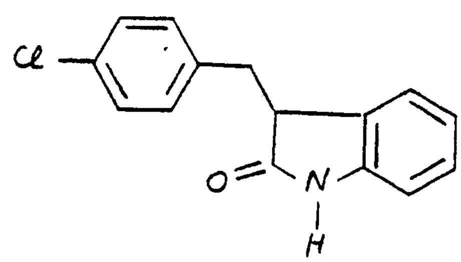

(D42)

A suspension of 0.5g (1.5mmol) ethyl 3-[(4-chlorophenyl)methyl]-2,3-dihydro-2-oxo-1H-indole-3-carboxylate (D41) in 7ml 47% aqueous hydrobromic acid was stirred for 0.5 hours at 50°C then 1 hour under reflux. The reaction mixture was poured onto ice then extracted with diethyl ether (2 x 50ml). The organic layer was washed with water until pH7 was reached, dried over $MgSO_4$ and concentrated to dryness. The residue was triturated in a mixture of brine and diisopropyl ether to afford the desired compound.
Yield : 0.3g (77.6%)
m.p. : 143°C

IR (KBr) : ν = 1,695; 1,620; 1,490; 1,470; 1,235 $cm^{-1}$.
NMR ($CDCl_3$) : δ = 3.00 (dd,J = 13.7Hz,J′ = 8.5Hz,1H,$CH_2$Ar); 3.41 (dd,J = 13.7Hz,Jα = 4.7Hz,1H,$CH_2$Ar); 3.73 (dd,J′ = 8.5Hz,Jα = 4.7Hz,1H,CH-$CH_2$Ar); 6.80 to 7.29 (m,8H,Ar); 8.37 (s,1H,NH).

Description 43

5-[(2-bromo-1-oxo)ethyl]-3-[(4-chlorophenyl)methyl]-1,3-dihydro-2H-indol-2-one

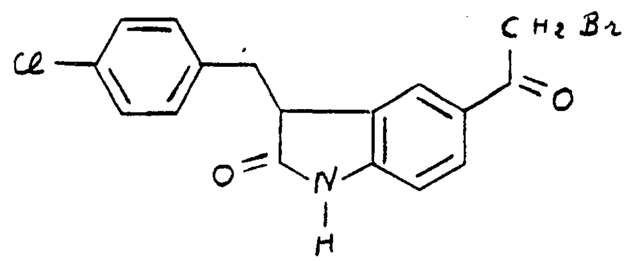

(D43)

0.25ml DMF was added dropwise to 1.5g (11mmol) aluminium chloride. The resulting mixture was heated for 5min at 50-60°C under stirring then cooled to room temperature. 0.28g (11mmol) 3-[(4-chlorophenyl)methyl]-1,3-dihydro-2H-indol-2-one (D42) then 0.17g (1.1mmol) bromacetyl chloride were added. After stirring for 1 hour at 40 to 50°C, the reaction mixture was poured onto ice water. The precipitate was filtered off, washed with water and dried to afford the desired compound which was used in the next step without further purification.
Yield : 0.34g (81.6%)
m.p. : 191°C
IR (KBr) : ν = 1,725; 1,695; 1,665; 1,615; 1,495; 1,100 $cm^{-1}$.
NMR (DMSO-$d_6$) : δ = 3.10 (dd,J = 13.7Hz,J′ = 6.6Hz,1H,$CH_2$Ar); 3.35 (dd,J = 13.7Hz,Jα = 5.0Hz,1H,$CH_2$Ar); 3.93 (dd,J′ = 6.6Hz,Jα = 5.0Hz,1H,CH-$CH_2$Ar); 4.81 and 4.75 (2d,J = 16.7Hz,2H,$CH_2$Br); 6.83 (d,J = 8.2Hz,1H,Ar); 7.13 (d,J = 8.3Hz,2H,Ar); 7.24 (d,J = 8.3Hz,2H,Ar); 7.74 (s,1H,Ar); 7.85 (d,J = 8.2Hz,1H,Ar); 10.78 (s,1H,NH).

Example 10

3-Cyclohexyl-1,3-dihydro-5-(3,6-dihydro-6-methyl-2-oxo-2H-1,3,4-thiadiazin-5-yl)-2H-indol-2-one

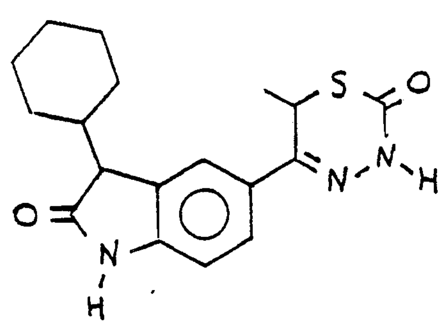

(E10)

1g 5-(2-bromo-1-oxopropyl)-3-cyclohexyl-1,3-dihydro-2H-indol-2-one (D19), 20ml acetonitrile, 450mg O-methyl thiocarbazide and 300mg triethylamine were refluxed for 3 hours.
Work up gave the desired compound.
Yield : 17%
m.p. : 155°C
IR (KBr) : $\nu$ = 3,250 ; 2,940 ; 1,685; 1,645; 1,620 $cm^{-1}$.
NMR $(DMSO-d_6)$ : $\delta$ = 1.0-2.1 (m,14H,cyclohexyl 11H, + d,1,47 ppm,J = 7.1Hz,3H,$CH_3$); 3.38-(d,1H,J = 7.0Hz,CH cyclohexyl); 4.76 (q,1H,J = 7.1Hz,$CHCH_3$); 6.87 (d,J = 8.1Hz,1H,Ar); 7.63 (d,J = 8.1Hz,1H,Ar); 7.73 (s,1H,Ar); 10.61 (s,1H,exch.$D_2O$,NH); 11.59 (s,1H,exch.$D_2O$,NH).

Example 11

1,3-Dihydro-5-(3,6-dihydro-2-oxo-2H-1,3,4-thiadiazin-5-yl)-3-phenylthio-2H-indol-2-one

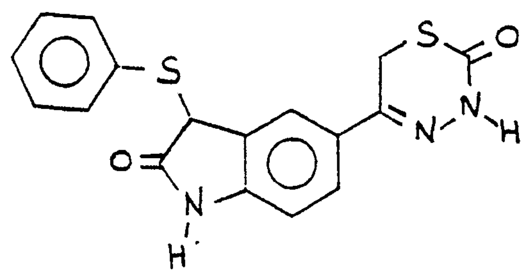

(E11)

Starting from 5-(2-chloro-1-oxoethyl)-1,3-dihydro-3-phenylthio-2H-indol-2-one D20 and using the method described in Example 2, gave the desired compound.
Yield : 67%
m.p. : 229°C
IR (KBr) : $\nu$ = 3,200 ; 1,702 ; 1,640; 1,610 $cm^{-1}$.
NMR $(DMSO-d_6)$ : $\delta$ = 4.19 (s,2H,$CH_2S$); 5.11 (s,1H CHS); 6.81 (d,1H,J = 8.2Hz,Ar indole); 7.26 (m,5H,ArS); 7.65 (d, 1H,J = 8.2Hz,Ar indole); 7.78 (s,1H,Ar indole); 10.75 (s,1H,exch.$D_2O$,NH); 11.53 (s,1H,exch.$D_2O$,NH).

Example 12

3-Cyclohexyl-1,3-dihydro-5-(3,6-dihydro-2-oxo-2H-1,3,4-thiadiazin-5-yl)-2H-indol-2-one

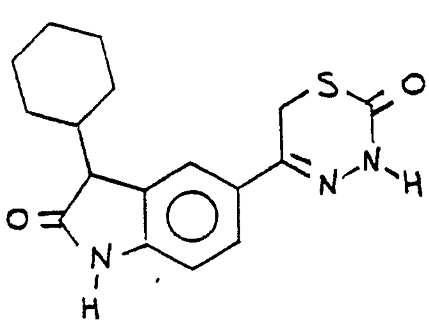

(E12)

Starting from 5-(2-chloro-1-oxoethyl)-3-cyclohexyl-1,3-dihydro-2H-indol-2-one (D21) and following the method described for Example 2, the desired compound was obtained.

Yield : 21%
m.p. : 258°C
IR (KBr) : $\nu$ = 3,200 ; 2,920 ; 1,715; 1,620cm$^{-1}$.
NMR (DMSO-$d_6$) : $\delta$ = 0.9-2.1 (m,11H,cyclohexyl); 3.39 (d,1H,J=2,5Hz,CH cyclohexyl); 4.20 (s,2H,$CH_2S$); 6.87 (d,1H,J=8.2Hz,Ar); 7.65 (d,1H,J=8.2Hz,Ar); 7.72 (s,1H,Ar); 10.59 (s,1H,exch.$D_2O$,NH); 11.49 (s,1H,exch.$D_2O$,NH).

Example 13

1,3-Dihydro-5-(3,6-dihydro-6-methyl-2-oxo-2H-1,3,4-thiadiazin-5-yl)-3-methyl-3-phenylthio-2H-indol-2-one

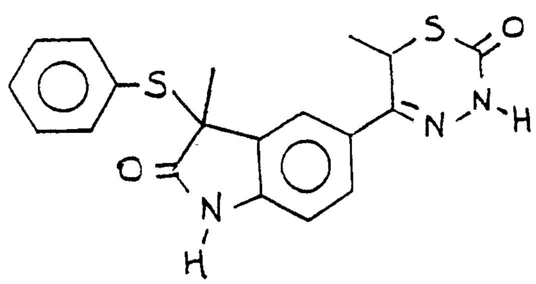

(E13)

Starting from 5-(2-chloro-1-oxopropyl)-1,3-dihydro-3-methyl-3-phenylthio-2H-indol-2-one (D22) and following the method given in Example 2, the desired compound was obtained.
Yield : 10%
m.p. : 115°C
IR (KBr) : $\nu$ = 3,200 ; 1,720 ; 1,620 cm$^{-1}$.
NMR (DMSO-$d_6$) : $\delta$ = 1.69 (d,3H,J=7.2Hz,$CH_3CH$); 1.74 (s,3H,$CH_3C$); 4.25 (q,1H,J=7.2Hz $CH_3CH$); 6.7-7.8 (m,8H,Ar); 8.27 (s,1H,exch.$D_2O$,NH); 9.14 (s,1H,exch.$D_2O$,NH).

Example 14

1,3-Dihydro-5-(3,6-dihydro-6-methyl-2-oxo-2H-1,3,4-thiadiazin-5-yl)-3,3-di-(4-methylphenyl)-2H-indol-2-one

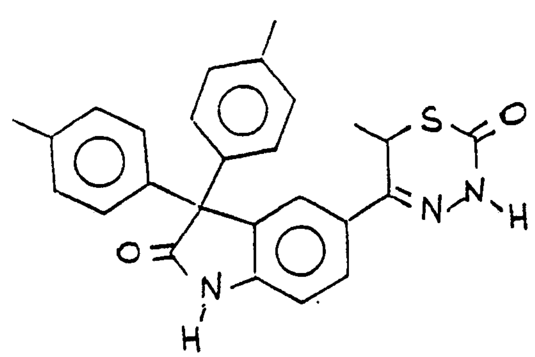

(E14)

Starting from 5-(2-bromo-1-oxopropyl)-1,3-dihydro-3,3-di-(4-methylphenyl)-2H-indol-2-one (D25) and following the method given in Example 2, the desired compound was obtained.
Yield : 61%
m.p. : 200°C
IR (KBr) : $\nu$ = 3,200 ; 1,710 ; 1,645; 1,615 cm$^{-1}$.

NMR (DMSO-$d_6$) : δ = 1.44 (d,3H,J=7.1Hz,$CH_3CH$); 2.26 (s,6H,2 x $CH_3Ar$); 4.71 (q,1H,J=7.1Hz,$CH_3CH$); 7.04 (d,1H,J 8.3Hz,Ar indole); 7.05 (d,4H,J=8.2Hz,Toluyl); 7.14 (d,4H,J=8.2Hz,Toluyl); 7.68 (s,1H,Ar indole); 7.72 (d,1H,J=8.3Hz,Ar indole); 10.98 (s,1H,exch.$D_2O$,NH); 11.55 (s,1H,exch.$D_2O$,NH).

Example 15

S-Ethyl 2,3-dihydro-5-(3,6-dihydro-6-methyl-2-oxo-1,3,4-thiadiazin-5-yl)-3,3-dimethyl-2-oxo-1H-indole-1-carbothioate

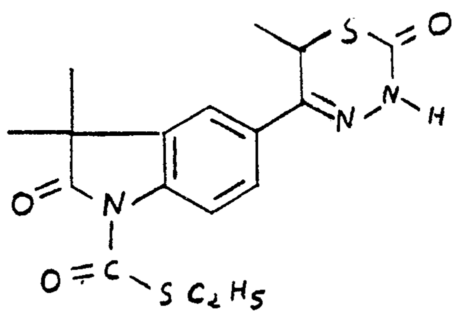

(E15)

4.95g (12.9mmol) S-ethyl -5-[(2-bromo-1-oxo)propyl]-2,3-dihydro-3,3-dimethyl-2-oxo-1H-indole-1-carbothioate (D27), 2.74g (25.8mmol)0-methyl thiocarbazate and 2.94g (25.8mmol) trifluoroacetic acid in 60ml acetonitrile were refluxed for 4 hours with stirring. The solvent was evaporated, the residue was triturated in water, dried and purified by chromatography on silica (methylene chloride/ethyl acetate : 98/2) to afford 3.0g (61%) of the title compound.
m.p. : 213°C
IR (KBr) : ν = 1,750 ; 1,630 ; 1,270 $cm^{-1}$.
NMR (DMSO-$d_6$) : δ = 1.29 (t,J=7.3Hz,3H,$CH_3$-$CH_2$); 1.43 (s,6H,$(CH_3)_2C$); 1.50 (d,J=7.1Hz,3H,$CH_3$-CH); 2.96 (q,J=7.3,2H,$CH_3$-$CH_2$); 4.78 (q,J=7.1Hz,1H,CH-$CH_3$); 7.81 (dd,J=1.5Hz,J′=8.6Hz,1H,Ar); 7.92 (d,J=1.5Hz,1H,Ar); 8.14 (d,J′=8.6Hz,1H,Ar); 11.72 (s,1H,NH).

Example 16

2,2,2-Trifluoroethyl 2,3-dihydro-5-(3,6-dihydro-6-methyl-2-oxo-1,3,4-thiadiazin-5-yl)-3,3-dimethyl-2-oxo-1H-indole-1-carboxylate

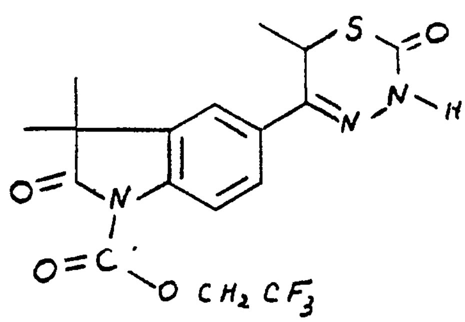

(E16)

2.1g (5mmol) 2,2,2-trifluoroethyl 5-[(2-bromo-1-oxo)propyl]-2,3-dihydro-3,3-dimethyl-2-oxo-1H-indole-1-carboxylate (D29), 0.60g (5.5mmol) 0-methyl thiocarbazate and 0.63g (5.5mmol) trifluoroacetic acid in 20ml acetonitrile were refluxed for 2.5 hours with stirring. The solvent was evaporated and the residue taken up in

150ml diethyl ether. The organic solution was washed with water (2 x 50ml), dried over $MgSO_4$ and evaporated to dryness. The resulting crude compound was purified by chromatography on silica (hexane/ethyl acetate : 1/1) then triturated in diethyl ether.
Yield : 0.95g (47.3%)
m.p. : 195°C
IR (KBr) : $\nu$ = 3,300 ; 1,790 ; 1,740; 1,635; 1,235; 1,170 $cm^{-1}$.
NMR ($CDCl_3$) : $\delta$ = 1.50 (s,6H,$(CH_3)_2C$); 1.70 (d,J=7.3Hz,3H,$CH_3$CH); 4.30 (q,J=7.3Hz,1H,$CH_3$CH); 4.79 (q,J=8.2Hz,2H,$CF_3CH_2$); 7.62 (dd,J′=1.7Hz,J=8.6Hz,1H,Ar);7.74 (d,J′=1.7Hz,1H,Ar); 7.94 (d,J=8.6Hz,1H,Ar); 8.94 (s,1H,NH).

Example 17

2-Methoxyethyl 2,3-dihydro-5-(3,6-dihydro-6-methyl-2-oxo-1,3,4-thiadiazin-5-yl)-3,3-dimethyl-2-oxo-1H-indole-1-carboxylate

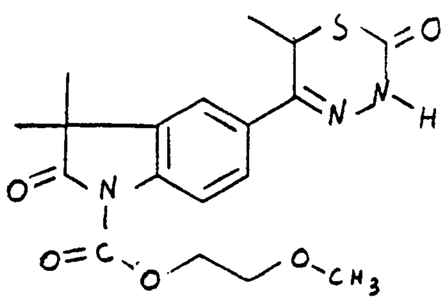

(E17)

4.1g (10.3mmol) 2-methoxyethyl 5-[(2-bromo-1-oxo)propyl]-2,3-dihydro-3,3-dimethyl-2-oxo-1H-indole-1-carboxylate (D31), 1.3g (12.3mmol) 0-methyl thiocarbazate and 1.3g (11.3mmol) trifluoroacetic acid in 50ml acetonitrile were refluxed with stirring for 3 hours. The solvent was evaporated and the residue taken up in water. The aqueous mixture was extracted with 150ml methylene chloride. The organic solution was washed with aqueous $NaHCO_3$ solution, with water (100ml) and dried over $MgSO_4$. The solvent was evaporated to dryness and the crude compound obtained was purified by chromatography on silica (methylene chloride/ethyl acetate : 9/1) then triturated in diisopropyl ether to afford the desired compound.
Yield : 2.76g (68.4%)
m.p. : 170°C
IR (KBr) : $\nu$ = 1,795; 1,630; 1,310; 1,240; 1,045 $cm^{-1}$.
NMR ($CDCl_3$) : $\delta$ = 1.48 (s,6H,$(CH_3)_2C$); 1.69 (d,J=7.3Hz,3H, $CH_3$-CH); 3.46 (s,3H,$OCH_3$); 3.78 (t,J=4.7Hz,2H,$CH_2$-$CH_2$); 4.30 (q,J=7.3Hz,1H,$CH_3$-CH); 4.57 (t,J=4.7Hz,2H,$CH_2$-$CH_2$); 7.59 (dd,J′=1.7Hz,J=8.6Hz,1H,Ar); 7.72 (d,J′=1.7Hz,1H,Ar); 7.98 (d,J=8.6Hz,1H,Ar); 8.94 (s,1H,NH).

Example 18

2-(Methylthio)ethyl 2,3-dihydro-5-(3,6-dihydro-6-methyl-2-oxo-1,3,4-thiadiazin-5-yl)-3,3-dimethyl-2-oxo-1H-indole-1-carboxylate

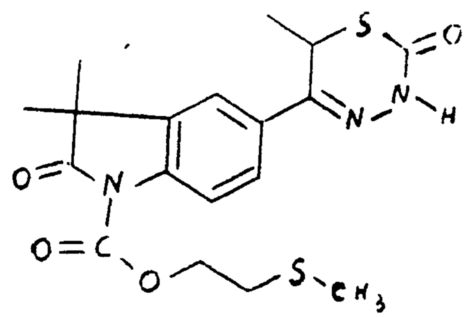

(E18)

Starting from 2-(methylthio)ethyl 5-[(2-bromo-1-oxo)propyl]-2,3-dihydro-3,3-dimethyl-2-oxo-1H-indole-1-carboxylate (D34), and O-methyl thiocarbazate and following the procedure of Example 17, the title compound was obtained after purification by chromatography on silica (methylene chloride/ethyl acetate : 9/1) then triturationin diisopropyl ether.
Yield : 40%
m.p. : 174°C
IR (KBr) : $\nu$ = 3,450 ; 1,790 ; 1,630; 1,310; 1,230 $cm^{-1}$.
NMR (DMSO-$d_6$) : $\delta$ = 1.40 (s,6H,$(CH_3)_2C$); 1.50 (d,J=7.1Hz,3H,$CH_3CH$); 2.16 (s,3H,$CH_3S$); 2.87 (t,J=6.6Hz,2H,$SCH_2CH_2$); 4.50 (t,J=6.6Hz,2H,$OCH_2$-$CH_2$); 4.78 (q,J=7.1Hz,1H,$CH_3CH$); 7.77 to 7.94 (m,3H,Ar); 11.70 (s,1H,NH).

Example 19

Ethyl 2,3-dihydro-5-(3,6-dihydro-6-methyl-2-oxo-1,3,4-thiadiazin-5-yl)-3,3-dimethyl-2-oxo-1H-indole-1-sulfinate

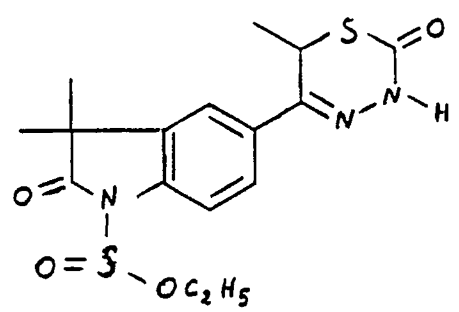

(E19)

Starting from ethyl 5-[(2-bromo-1-oxo)-propyl]-2,3-dihydro-3,3-dimethyl-2-oxo-1H-indole-1-sulfinate (D36), and O-methyl thiocarbazate and following the procedure of Example 15 with a reaction time of 2 hours instead of 4 hours, the title compound was obtained after purification by chromatography on silica (methylene chloride/ethyl acetate : 95/5).
Yield : 53.3%
m.p. : 185.5°C
IR (KBr) : $\nu$ = 3,450 ; 1,760 ; 1,630; 1,370; 1,160 $cm^{-1}$.
NMR (DMSO-$d_6$) : $\delta$ = 1.26 (t,J=7.3Hz,3H,$CH_3CH_2O$); 1.43 (s,6H,$(CH_3)_2C$); 1.50 (d,J=7.1Hz,3H,$CH_3$-CH); 3.72 (q,J=7.3Hz,2H,$CH_3CH_2O$); 4.76 (q,J=7.1Hz,1H,$CH_3$-CHS); 7.70 (d,J=8.6Hz,1H,Ar); 7.81 (dd,J=8.6Hz,J′=1.6Hz,1H,Ar); 7.93 (d,J′=1.6Hz,1H,Ar); 11.71 (s,1H,NH).

Example 20

Cyclohexyl 2,3-dihydro-5-(3,6-dihydro-6-methyl-2-oxo-1,3,4-thiadiazin-5-yl)-3,3-dimethyl-2-oxo-1H-indole-1-carboxylate

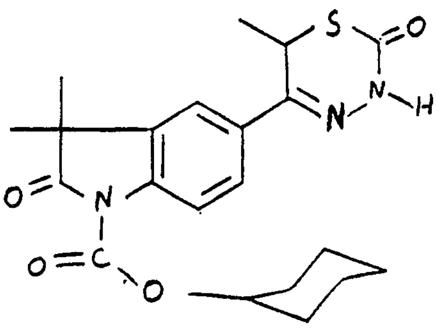

(E20)

2.0g (4.7mmol) cyclohexyl 5-[(2-bromo-1-oxo)-propyl]-2,3-dihydro-3,3-dimethyl-2-oxo-1H-indole-1-carboxylate (D38), 0.53g (4.9mmol) O-methyl thiocarbazate and 0.54g (4.7mmol) trifluoroacetic acid in 24ml acetonitrile were refluxed for 3 hours with stirring. The solvent was evaporated under vacuum and the resulting residue taken up in methylene chloride. The organic solution was washed with water, with aqueous $NaHCO_3$ solution, again with water, and dried over $MgSO_4$. The solvent was evaporated to dryness and the crude compound obtained was purified by chromatography on silica (methylene chloride/ethyl acetate : 95/5) then triturated in ligroin to afford the desired compound.

Yield : 1.25g (64%)

m.p. : 206-7°C

IR (KBr) : $\nu$ = 1,780 ; 1,720 ; 1,630; 1,240 $cm^{-1}$.

NMR ($CDCl_3$) : $\delta$ = 1.40 to 2.05 (m,19H,-$(CH_2)_5$-(10H) and including s at 1.47 ppm,6H,$(CH_3)_2C$ and d at 1.69 ppm, J=7.2Hz,3H,$CH_3CH$); 4.30 (q,J=7.2Hz,1H,$CH_3CHS$); 5.06 (m,1H,OCH); 7.59 (dd,J=8.6Hz,J′=1.8Hz,1H,Ar); 7.71 (d,J′=1.8Hz,1H,Ar); 7.97 (d,J=8.6Hz,1h,Ar); 8.92 (s,1H,NS).

Example 21

2-Cyanoethyl 2,3-dihydro-5-(3,6-dihydro-6-methyl-2-oxo-1,3,4-thiadiazin-5-yl)-3,3-dimethyl-2-oxo-1H-indole-1-carboxylate

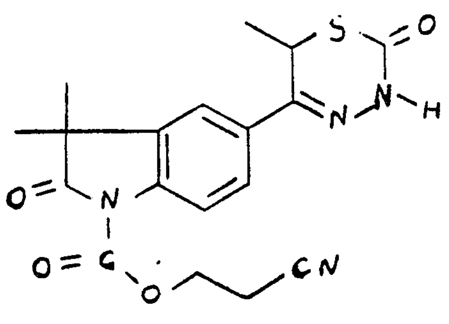

(E21)

1.2g (3.05mmol) 2-cyanoethyl 5-[(2-bromo-1-oxo)-propyl]-2,3-dihydro-3,3-dimethyl-2-oxo-1H-indole-1-carboxylate (D40),0.34g (3.2mmol) O-methyl thiocarbazate and 0.71g (6.2mmol) trifluoroacetic acid in 16ml acetonitrile were refluxed for 3 hours with stirring. The solvent was evaporated and the residue taken up in 150ml methylene chloride. The organic solution was washed with water diluted $NaHCO_3$, again with water, dried over $MgSO_4$ and evaporated to dryness. The residue was purified twice by chromatography on silica (methylene chloride/ethyl acetate : 9/1) and (methylene chloride/diethyl ether : 9/1) to afford the desired

compound.
Yield : 0.58g (50%)
m.p. : 184-5°C
IR (KBr) : $\nu$ = 3,450 ; 2,250 ; 1,710; 1,640; 1,620; 1,390, 1,235 $cm^{-1}$.
NMR ($CDCl_3$) : $\delta$ = 1.43 (s,6H,$(CH_3)_2C$); 1.69 (d,J=7.2Hz,3H,$CH_3$-CH); 2.80 (t,J=6.8Hz,2H,$CH_2CN$); 4.05 (t,J=6.8Hz,2H,$OCH_2$); 4.28 (q,J=7.2Hz,1H,$CH_3$-CH); 6.99 (d,J=8.3Hz,1H,Ar); 7.59 (dd,J=8.3Hz,J′=1.7Hz,1H,Ar); 7.73 (d,J′=1.7Hz,1H,Ar); 8.92 (s,1H,NH).

Example 22

Diethyl 2,3-dihydro-5-(3,6-dihydro-6-methyl-2-oxo-1,3,4-thiadiazin-5-yl)-3,3-dimethyl-2-oxo-1H-indole-1-phosphonate

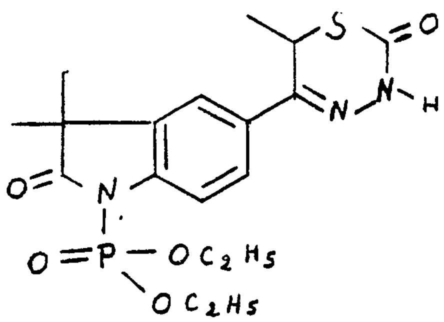

(E22)

4g (13.8mmol) 1,3-dihydro-5-(3,6-dihydro-6-methyl)-2-oxo-2H-1,3,4-thiadiazin-5-yl)-3,3-dimethyl-2H-indol-2-one (Laboratoires Sobio EP-A-0,303,418) in 60ml DMF were cooled at 0°C. 0.61g (13.9mmol) sodium hydride from a 55% dispersion in mineral oil was added and the mixture was stirred for 0.5 hours at 0-5°C then at room temperature until hydrogen evolution ceased. After cooling to 0°C, 2.4g (13.9mmol) diethyl chlorophosphate were added dropwise. The reaction mixture was stirred at room temperature for 16 hours then the solvent was evaporated under vacuum. The residue was taken up in methylene chloride, washed with water and dried over $CaCl_2$. The solvent was evaporated and the crude compound obtained was purified by chromatography on silica (methylene chloride/ethyl acetate : 8/2) then trituration in diisopropyl ether.
Yield : 1.27g (22%)
m.p. : 140°C
IR (KBr) : $\nu$ = 1,740 ; 1,660 ; 1,620; 1,495; 1,260; 1,140; 1,030 $cm^{-1}$.
NMR (DMSO-$d_6$) : $\delta$ = 1.25 (t,J=7.0Hz,6H,$2CH_3CH_2O$); 1.39 (s,6H,$(CH_3)_2C$); 1.50 (d,J=7.1Hz,3H,$CH_3CH$); 4.06 to 4.27 (2q,J=7.0Hz,4H,$2CH_3CH_2O$); 4.75 (q,J=7.1Hz,1H,$CH_3$-CH); 7.77 to 7.89 (m,3H,Ar); 11.67 (s,1H,NH).

Example 23

2-Propenyl 2,3-dihydro-5-(3,6-dihydro-6-methyl-2-oxo-1,3,4-thiadiazin-5-yl)-3,3-dimethyl-2-oxo-1H-indole-1-carboxylate

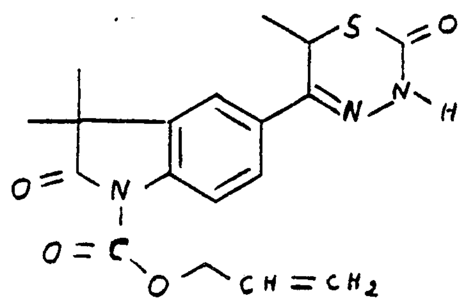

(E23)

Starting from 1,3-dihydro-5-(3,6-dihydro-6-methyl)-2-oxo-2H-1,3,4-thiadiazin-5-yl)-3,3-dimethyl-2H-indol-2-one and 2-propene chloroformate and following the procedure of Example 22 with a reaction time of 2 hours instead of 16 hours, the title compound was obtained after purification by chromatography on silica (methylene chloride/ethyl acetate : 9/1).

Yield : 45.6%

m.p. : 194°C

IR (KBr) : $\nu$ = 1,760 ; 1,730 ; 1,655; 1,620; 1,300; 1,280; 1,230 $cm^{-1}$.

NMR ($CDCl_3$) : $\delta$ = 1.48 (s,6H,$(CH_3)_2C$); 1.69 (d,J:7.2Hz,3H, $CH_3CH$); 4.30 (q,H=7.2Hz,1H,$CH_3$-CH); 4.92 (d,J=5.6Hz,2H,$OCH_2$-CH=); 4.92 (dd,Jcis=10.3Hz,Jgem=1.3Hz,1H,$CH_2$=CH-); 5.56 (dd,Jtrans=17.2Hz,Jgem=1.3Hz,1H,$CH_2$=CH-); 6.03 (m,2H,$OCH_2$-CH=$CH_2$); 7.59 (q,J=8.5Hz,J′=1.8Hz,1H,Ar); 7.72 (d,J′=1.8Hz,1H,Ar); 8.00 (d,J:8.5Hz,1H,Ar); 8.97 (s,1H,NH).

Example 24

3-[(4-Chlorophenyl)methyl]-1,3-dihydro-5-(3,6-dihydro-2-oxo-2H-1,3,4-thiadiazin-5-yl)-2H-indol-2-one

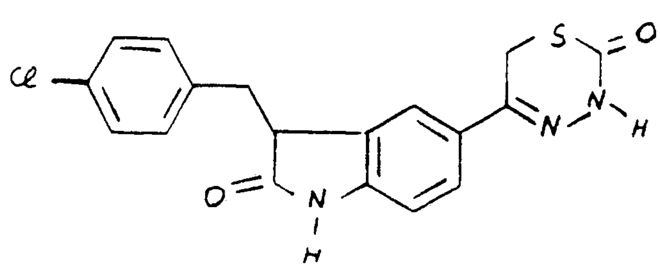

(E24)

0.3g (0.8mmol)5-[(2-bromo-1-oxo)ethyl]-3-[(-4 chlorophenyl)methyl]-1,3-dihydro-2H-indol-2-one) (D43) and 0.1g (0.9mmol) O-methyl thiocarbazate in 2.5ml acetonitrile were refluxed for 3 hours. On cooling at room temperature, the compound crystallized : it was filtered off, washed with acetonitrile and dried to afford the title compound.

Yield : 0.21g (70.6%)

m.p. : 267°C

IR (KBr) : $\nu$ = 1,690 ; 1,655 ; 1,620; 1,495; 1,240; 1,190 $cm^{-1}$.

NMR (DMSO-$d_6$) : $\delta$ = 3.13 (dd,J=13.8Hz,J′=6.2Hz,1H,$CH_2$Ar); 3.30 (dd,J=13.8Hz,Jα=5.3Hz,1H,$CH_2$Ar); 3.38 (dd,J′=6.2Hz, Jα=5.3Hz,1H,CH-$CH_2$Ar); 4.15 (s,2H,$CH_2$S); 6.78 (d,J=8.1Hz,1H,Ar); 7.10 (d,J=8.4Hz,2H,Ar); 7.24 (d,J=8.4Hz,2H,Ar); 7.60 (d,J=8.1Hz,1H,Ar); 7.62 (s,1H,Ar); 10.54 (s,1H,NH); 11.49 (s,1H,NH).

Pharmacological Data

1. Measurement of myofibrillar Ca++-dependent Mg++-ATPase activity

The effect on Ca++sensitivity and on maximal activity of myofibrillar Ca++-dependent Mg++-ATPase was determined on canine cardiac myofibrils.

(a) Contractile protein preparation

Cardiac myofibrils free of membrane contaminants were prepared from left ventricular tissue of dog hearts using a modification of the method described by SOLARO et al, Biochem. Biophys. Acta, 245, 259-262, (1971). Before centrifugation, the heart homogenate was filtered through gauze.

After Triton X-100 treatments, the myofibrillar fraction was washed once by resuspension and centrifugation in 10 volumes of 1 mM EGTA, 60ml KCl, 30 mM imidazole, 2 mM $MgCl_2$, pH 7.0. The pellet was then washed three times in 10 volumes of the same buffer without EGTA. Before the last centrifugation, the suspension of myofibrils was filtered through a stainless steel sieve with 0.25 mm meshes.

Myofibrils were kept in pellet overnight. Before use, the pellet was suspended in a small amount of buffer, the protein concentration was determined by the method of BRADFORD M., Anal. Biochem., 1976, 72, 248 and adjusted in order to have a concentration between 6 and 7mg/ml, using gamma globuline as standard.

(b) Myofibrillar ATPase activity

Ca++-dependent myfibrillar ATPase activity was determined at 21°C, pH 7.0, by measuring the rate of release of inorganic phosphate. All assays were performed within 24hrs. of final purification, using the method described by SOLARO and RUEGG, CIRC. RES., 51, 290-4,(1982). Reaction mixtures (4ml) containing 0.6 - 0.7 mg/ml myofibrillar protein, 80mM KCl, 20 mM imidazole, 3 mM $MgCl_2$, 1 mM EGTA, the desired amount of $CaCl_2$, and the indicated concentration of drug or appropriate vehicle were preincubated for 6 min. prior to assay. The amount of $CaCl_2$ was varied between 0 and 0.9 mM and the pca (-log free Ca++) computed using $2.514 \times 10^6$ $M^{-1}$ as the apparent affinity of Ca++ for EGTA at pH 7.0.

Reactions were initiated by the addition of $Na_2ATP$ to final concentration of 2 mM. After an incubation period of 12 min., reactions were quenched by the addition of an equal volume of ice-cold 10% trichloroacetic acid. The protein was pelleted by centrifugation (2000g, 15 min.) and Pi was assayed in the supernatant fraction using a modification of the method described by LANZETTA et al, Anal. Biochem., 100, 95-97, (1979).

The following solutions were prepared:

(1) 3.7% ammonium heptamolybdate in 0.12 N $H_2SO_4$

(2) 0.074% malachite green oxalate in 1% polyvinylic alcohol.

The colour reagent was prepared by mixing an equal volume of solution (1) and (2) 15 min. before the assay. For the assay, 1700 µl of 1% $H_2SO_4$ and 1 ml of colour reagent were added to 100 µl of sample. Colour development occurred at room temperature for 30 min. and was quenched by the addition of 200 µl of 1 M tri-potassium citrate. The absorbance was measured at 620 millimicrons.

The effect of each compound, tested at the indicated concentration, on the relation between pCa and percent activation was determined taking 100% for the maximum ATPase activity obtained with vehicle alone. The effect on Ca++ sensitivity was quantified by measuring the shift of the pCa giving 50% of the maximal control ATPase activity.

The effect on maximal ATPase activity was expressed as the percent change of ATPase activity for pCa = 5.47.

2. Inhibition of Cardiac Phosphodiesterases

The phosphodiesterases (PDE) isoforms of canine left ventricle were separated by column ion exchange chromatography, using a minor modification of the method previously described by Alvarez et al. (Mol. Pharmacol., 1986, 29, 554-560). A Fractogel TSK DEAE 650 column was used and the PDEs were eluted using a continuous sodium acetate gradient (50 to 500 mM). The flow rate was 1.2ml/min and fractions of c.a. 8ml were collected as described by Weishaar et al. (Biochem. Pharmacol., 1986, 35, 787-800).

Four isoforms were identified

i) Fraction I or CAM-PDE (i.e. $Ca^{++}$/calmodulin-activated PDE), which hydrolyses both c-AMP and c-GMP, and presents a low Km for c-AMP over a wide range of concentration. It is activated by $Ca^{++}$/calmodulin.

ii) Fraction II or CGS-PDE (i.e. c-GMP-stimulated PDE), which hydrolyses more specifically c-GMP, and presents a high Km, for c-AMP over a wide range of concentration. It is stimulated by c-GMP.

iii) Fraction III or NCGI-PDE (i.e. not c-GMP-inhibited PDE), which hydrolyses more specifically c-AMP and presents two relatively low Km sites of affinity. It is not inhibited by c-GMP. iv) Fraction IV or CGI-PDE (i.e., c-GMP-inhibited PDE), which also hydrolyses more specifically c-AMP, and also presents two relatively low Km site of affinity. It is inhibited by c-GMP.

The inhibitory effect of the test compounds was expressed as the $IC_{50}$, i.e. the concentration which inhibits the activity by 50%, this value being expressed in micromoles.

| Results | | | |
|---|---|---|---|
| Canine cardiac myofibrillar $Ca^{++}$-dependent $Mg^{++}$ ATPase. (Concentration: $3 \times 10^{-5}$ molar) | | | |
| Example No. | $Ca^{++}$ sensitivity $pCA_{50}$ | Max ATPase activity(%) | n |
| 1 | 0.19 | -18 | 2 |
| 3 | 0.23 | - 6 | 2 |
| 4 | 0.68 | - 6 | 3 |
| 5 | 0.38 | + 2 | 1 |
| 7 | 0.20 | + 4 | 1 |
| 9 | 0.32 | +11 | 1 |
| 10 | 0.99 | + 1 | 3 |
| 11 | 0.21 | + 5 | 3 |
| 12 | 0.49 | - 2 | 2 |

3. Sarcoplasmic reticulum phosphodiesterase (SR-PDE):

Sarcoplasmic reticulum (SR) vesicles, A-E, were prepared from ventricles of pentobarbital anaesthetized dogs as described by Jones et al (1,2). Fraction E, which originates from free SR vesicles (3), was used for the SR-PDE inhibition study. Aliquots of free SR vesicles were used within 48 hours following their preparation.

References:

1. Jones, L.R. Besch, H.R., Fleming J.W. McConnaughey, M.M., Watanabe A.M., J. Biol. Chem., 1979, 254, 530.

2. Jones, L.R. , Cala, S.E. J. Biol. Chem., 1981, 256, 11809.

3. Seiler S., Wegener A.D. Whang, D.D., Hathaway D.R., Jones L.R., J. Biol. Chem. 1984, 259, 8550.

<table>
<tr><th colspan="4">PDE inhibition results</th></tr>
<tr><th>Example</th><th>CAM</th><th>CGI</th><th>SR-PDE</th></tr>
<tr><td>1<br>2<br>3<br>4</td><td>(28*)<br>( 4*)<br>230<br>(37*)</td><td>0.87<br>15<br>6.7<br>3.2</td><td>not tested<br>not tested<br>not tested<br>not tested</td></tr>
<tr><td>5</td><td colspan="2">not tested</td><td>0.007</td></tr>
<tr><td>7</td><td colspan="2">not tested</td><td>0.029</td></tr>
<tr><td>8</td><td colspan="2">not tested</td><td>2.6</td></tr>
</table>

* Maximal inhibition not reached up to 300μM.

## Claims

1. A compound of formula (I), or a pharmaceutically acceptable salt thereof:

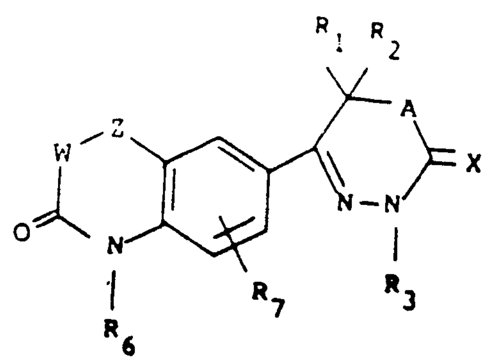

(I)

in which,
$R_1$ is hydrogen, $C_{1-6}$ alkyl or $CH_2OR_6$;
$R_2$ is hydrogen or $C_{1-6}$ alkyl;
$R_3$ is hydrogen or $C_{1-6}$ alkyl;
each of W and Z, which are different, represents $-CR_4R_5-$ or $-(CR_xR_y)_n-$, in which,
$R_4$ is hydrogen, $C_{1-3}$ alkyl, $C_{1-3}$ alkylthio, $C_{1-3}$ alkoxy or $C_{1-6}$ alkyl phenyl;
$R_5$ is $C_{1-3}$ alkyl, $C_{1-3}$ alkylthio, $C_{1-3}$ alkoxy, phenyl, substituted phenyl, $C_{3-6}$ cycloalkyl, phenylthio, $C_{1-6}$ alkyl phenyl, halo-substituted benzyl, or heteroaryl; or together $R_4$ and $R_5$ form a 3 to 6 membered carbocyclic ring, or a heterocyclic ring containing one or two ring oxygen, nitrogen or sulphur atoms, or $R_4$ and $R_5$ together form an oxo or methylene group;
each of $R_x$ and $R_y$ is hydrogen or $C_{1-3}$ alkyl; n is zero or 1;
$R_6$ is phenyl substituted aminocarbonyl, $C_{1-6}$ alkoxy carbonyl-$C_{1-6}$ alkyl, phenyl-$C_{1-6}$ alkyl, phenyl, $C_{3-6}$ cycloalkylcarbonyl, $C_{3-6}$ cycloalkylcarbonyl-$C_{1-6}$ alkyl, $C_{3-6}$ cycloalkyl $C_{1-6}$ alkyl; $C_{1-6}$ alkylthiocarbonyl; halo-substituted $C_{1-6}$ alkoxycarbonyl; $C_{1-6}$ alkoxy $C_{1-6}$ alkyleneoxycarbonyl; $C_{1-6}$ alkylthio $C_{1-6}$ alkyleneoxycarbonyl; $C_{1-6}$ alkoxythiocarbonyl; $C_{3-6}$ cycloalkyloxycarbonyl; cyano substituted $C_{1-6}$ alkoxycarbonyl; di-$C_{1-6}$ alkylphosphonate; $C_{1-6}$ alkenyloxycarbonyl; or
$R_6$ is hydrogen when $R_5$ is phenyl, $C_{3-6}$ cycloalkyl, phenylthio, $C_{1-6}$ alkylphenyl or halo-substituted benzyl;
$R_6$ is benzoyl or aminobenzoyl when $R_4$ and $R_5$ form a $C_{3-6}$ cycloalkyl ring;
$R_7$ is hydrogen, $C_{1-6}$ alkyl or halogen;
X is oxygen or sulphur;
and A is sulphur, oxygen or -NH-.

2. A compound according to claim 1, in which n is zero.

3. A compound according to claim 1 or 2, in which A is sulphur.

4. A compound according to any one of claims 1 to 3, in which X is oxygen.

5. A compound according to any one of claims 1 to 4 in which,

$R_1$ is hydrogen or methyl;

$R_2$ is hydrogen or methyl;

$R_3$ is hydrogen or methyl;

$R_4$ is hydrogen or methyl;

$R_5$ is methyl or phenyl; or

$R_4$ and $R_5$ together are cyclopropyl or cyclopentyl;

$R_6$ is hydrogen, p-aminobenzoyl, phenylaminocarbonyl, ethoxycarbonylmethyl, benzyl, benzoyl, phenyl, phenyl-ethyl, or cyclohexyl-methyl;

and $R_7$ is hydrogen.

6. A compound selected from:

1′-[(4-Aminophenyl)carbonyl]-5′-(3,6-dihydro-6-methyl-2 oxo-2H-1,3,4-thiadiazin-5-yl)-spiro[cyclopentane-1,3′-[3H]-indol]-2′(1H)-one;

2,3-Dihydro-5-(3,6-dihydro-6-methyl-2-oxo-2H-1,3,4-thiadiazin-5-yl)-3,3-dimethyl-2-oxo-N-phenyl-1H-indole-1-carboxamide;

Ethyl 2,3-dihydro-5-(3,6-dihydro-6-methyl-2-oxo-2H-1,3,4-thiadiazin-5-yl)-3,3-dimethyl-2-oxo-N-1H-indole-1-acetate;

1,3-Dihydro-5-(3,6-dihydro-6-methyl-2-oxo-2H-1,3,4-thiadiazin-5-yl)-3-phenyl-2H-indol-2-one;

1,3-Dihydro-5-(3,6-dihydro-6-methyl-2-oxo-2H-1,3,4-thiadiazin-5-yl)-3,3-dimethyl-1-(phenylmethyl)-2H-indol 2-one;

1′-Benzoyl-5′-(3,6-dihydro-6-methyl-2-oxo-2H-1,3,4-thiadiazin-5-yl)-spiro[cyclopropane-1,3′-[3H]indol]-2′(1H)-one;

1,3-Dihydro-5-(3,6-dihydro-6-methyl-2-oxo-2H-1,3,4-thiadiazin-5-yl)-3,3-dimethyl-1-phenyl-2H-indol-2-one;

1,3-Dihydro-5-(3,6-dihydro-6-methyl-2-oxo-2H-1,3,4-thiadiazin-5-yl)-3,3-dimethyl-1-(2-phenylethyl)-2H-indol-2-one;

1-Cyclohexylmethyl-5-(3,6-dihydro-6-methyl-2-oxo-2H-1,3,4-thiadiazin-5-yl)-1,3-dihydro-3,3-dimethyl-2H-indol-2-one;

3-Cyclohexyl-1,3-dihydro-5-(3,6-dihydro-6-methyl-2-oxo-2H-1,3,4-thiadiazin-5-yl)-2H-indol-2-one;

1,3-Dihydro-5-(3,6-dihydro-2-oxo-2H-1,3,4-thiadiazin-5-yl)-3-phenylthio-2H-indol-2-one;

3-Cyclohexyl-1,3-dihydro-5-(3,6-dihydro-2-oxo-2H-1,3,4-thiadiazin-5-yl)-2H-indol-2-one;

1,3-Dihydro-5-(3,6-dihydro-6-methyl-2-oxo-2H-1,3,4-thiadiazin-5-yl)-3-methyl-3-phenylthio-2H-indol-2-one;

1,3-Dihydro-5-(3,6-dihydro-6-methyl-2-oxo-2H-1,3,4-thiadiazin-5-yl)-3,3-di-(4-methylphenyl)-2H-indol-2-one;

S-Ethyl 2,3-dihydro-5-(3,6-dihydro-6-methyl-2-oxo-1,3,4-thiadiazin-5-yl)-3,3-dimethyl-2-oxo-1H-indole-1-carbothioate;

2,2,2-Trifluoroethyl 2,3-dihydro-5-(3,6-dihydro-6-methyl-2-oxo-1,3,4-thiadiazin-5-yl)-3,3-dimethyl-2-oxo-1H-indole-1-carboxylate;

2-Methoxyethyl 2,3-dihydro-5-(3,6-dihydro-6-methyl-2-oxo-1,3,4-thiadiazin-5-yl)-3,3-dimethyl-2-oxo-1H-indole-1-carboxylate;

2-(Methylthio)ethyl 2,3-dihydro-5-(3,6-dihydro-6-methyl- 2-oxo-1,3,4-thiadiazin-5-yl)-3,3-dimethyl-2-oxo-1H-indole-1-carboxylate;

Ethyl 2,3-dihydro-5-(3,6-dihydro-6-methyl-2-oxo-1,3,4-thiadiazin-5-yl)-3,3-dimethyl-2-oxo-1H-indole-1-sulfinate;

Cyclohexyl 2,3-dihydro-5-(3,6-dihydro-6-methyl-2-oxo-1,3,4-thiadiazin-5-yl)-3,3-dimethyl-2-oxo-1H-indole-1-carboxylate;

2-Cyanoethyl 2,3-dihydro-5-(3,6-dihydro-6-methyl-2-oxo-1,3,4-thiadiazin-5-yl)-3,3-dimethyl-2-oxo-1H-indole-1-carboxylate;

Diethyl 2,3-dihydro-5-(3,6-dihydro-6-methyl-2-oxo-1,3,4-thiadiazin-5-yl)-3,3-dimethyl-2-oxo-1H-indole-1-phosphonate;

2-Propenyl 2,3-dihydro-5-(3,6-dihydro-6-methyl-2-oxo-1,3,4-thiadiazin-5-yl)-3,3-dimethyl-2-oxo-1H-indole-1-carboxylate;

3-[(4-Chlorophenyl)methyl]-1,3-dihydro-5-(3,6-dihydro-2-oxo-2H-1,3,4-thiadiazin-5-yl)-2H-indol-2-one.

7. A process for the preparation of a compound of formula (I), which comprises

(a) treating a compound of formula (II)

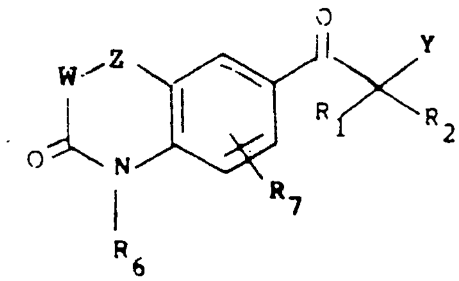

(II)

in which $R_1$, $R_2$, $R_6$, $R_7$, W and Z are as defined in formula (I), and
Y is a leaving group,
with a compound of formula (III)

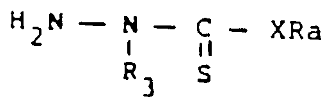

(III)

in which $R_3$ and X are as defined in formula (I), and $R_a$ is an alkyl group, and thereafter optionally converting a compound wherein X is oxygen to a compound wherein X is sulphur, or
(b) cyclising a compound of formula (IV):

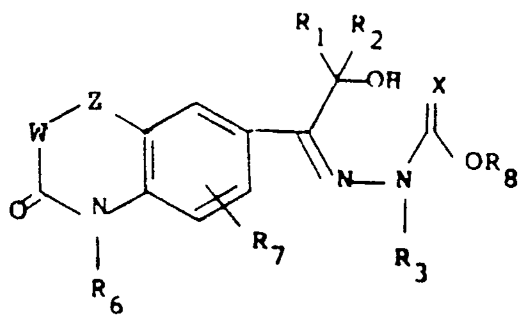

(IV)

in which $R_1$, $R_2$, $R_6$, $R_7$, X, W and Z are as defined in formula (I), and $R_8$ is an alkyl group, or
(c) treating a compound of formula (V):

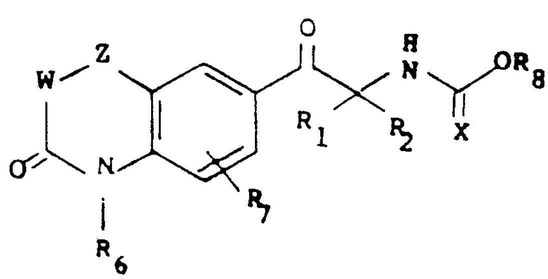

(V)

in which $R_1$, $R_2$, $R_6$, $R_7$, X, W and Z are as defined in formula (I) and $R_8$ is as defined in formula (IV), with a compound of formula (VI):
$H_2N\text{-}NH\text{-}R_3$ (VI)
in which $R_3$ is as defined in formula (I), and
optionally after steps (a), (b) or (c), converting a compound of formula (I) thereby produced to a pharmaceutically acceptable salt thereof or to a further compound of formula (I).

8. A pharmaceutical composition comprising a compound of formula (I) as defined in claim 1, or a pharmaceutically acceptable salt thereof, and a pharmaceutically acceptable carrier.

9. A compound of formula (I) or a pharmaceutically acceptable salt thereof, for use as an active therapeutic substance.

10. The use of a compound of formula I as defined in claim 1, or a pharmaceutically acceptable salt thereof, in the manufacture of a medicament for the treatment of heart disease or asthmatic conditions.

European Patent Office

# EUROPEAN SEARCH REPORT

Application number

EP 90300778.9

## DOCUMENTS CONSIDERED TO BE RELEVANT

| Category | Citation of document with indication, where appropriate, of relevant passages | Relevant to claim | CLASSIFICATION OF THE APPLICATION (Int Cl.5) |
|---|---|---|---|
| P,A | EP - A2 - 0 303 418 (LABAROATOIR SOBIO) * Claims 1-7 * | 1-10 | C 07 D 417/04<br>C 07 D 413/04<br>C 07 D 403/04<br>A 61 K 31/535<br>A 61 K 31/53<br>A 61 K 31/54 |
| A | EP - A2 - 0 294 647 (MERCK) * Claims 1-7 * | 1-10 | |
| A | EP - A1 - 0 180 158 (YOSHITOMI) * Claims 2,3 * | 1,10 | |
| A | EP - A2 - 0 220 044 (SMITH) * Formula I * | 1 | |

TECHNICAL FIELDS SEARCHED (Int Cl.5)

C 07 D 417/00
C 07 D 413/00
C 07 D 403/00

The present search report has been drawn up for all claims

| Place of search | Date of completion of the search | Examiner |
|---|---|---|
| VIENNA | 02-04-1990 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document